(19) Europäisches Patentamt
European Patent Office
Office européen des brevets

(11) Publication number: **0 077 109 B1**

# (12) EUROPEAN PATENT SPECIFICATION

(45) Date of publication of patent specification: **23.08.89**

(51) Int. Cl.[4]: **C 12 N 15/00**, C 12 N 9/52, C 12 N 9/60, C 12 N 1/00, C 12 R 1/19, C 12 R 1/46, C 12 R 1/225, C 12 R 1/07, C 12 R 1/465

(21) Application number: **82201272.0**

(22) Date of filing: **13.10.82**

## (54) DNA molecules comprising the genes for preprochymosin and its maturation forms, and microorganisms transformed thereby.

(30) Priority: **14.10.81 GB 8131004**

(43) Date of publication of application: **20.04.83 Bulletin 83/16**

(45) Publication of the grant of the patent: **23.08.89 Bulletin 89/34**

(84) Designated Contracting States: **AT BE CH DE FR GB IT LI NL SE**

(56) References cited:
**EP-A-0 036 776**
**EP-A-0 054 330**
**EP-A-0 057 350**
**AGRIC. BIOL. CHEM., vol. 44, no. 6, 1980, pages 1373-1381; H. UCHIYAMA et al.: "Purification of prorennin mRNA and its translation in Vitro"**
**JOURNAL OF BIOCHEMISTRY, vol. 90, no. 3, 1981, pages 901-904; K. NISHIMORI et al.: "Cloning in Escherichia coli of the structural gene of prorennin, the precursor of calf milk-clotting enzyme rennin"**

(73) Proprietor: **UNILEVER NV**
**Burgemeester s'Jacobplein 1 P.O. Box 760**
**NL-3000 DK Rotterdam (NL)**
(84) **BE CH DE FR IT LI NL SE AT**

(73) Proprietor: **UNILEVER PLC**
**Unilever House Blackfriars P.O. Box 68**
**London EC4P 4BQ (GB)**
(84) **GB**

(72) Inventor: **Maat, Jan**
**Molenstraat 2**
**NL-2681 BS Monster (NL)**
Inventor: **Verrips, Cornelis Theodorus**
**Hagedoorn 18**
**NL-3142 KB Maassluis (NL)**
Inventor: **Ledeboer, Adrianus Marinus**
**Burg. Le Fèvre de Montignyplein 8**
**NL-3055 NL Rotterdam (NL)**
Inventor: **Edens, Luppo**
**Albert Schweitzerdreef 29**
**NL-3146 AA Maassluis (NL)**

(74) Representative: **van der Toorren, Johannes, Drs. et al**
**UNILEVER N.V. Patent Division Postbus 137**
**NL-3130 AC Vlaardingen (NL)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

(56) References cited:

J. BIOCHEMISTRY, vol. 91, no. 3, 1982, pages 1085-1088; K. NISHIMORI et al.: "Nucleotide sequence of calf prorennin cDNA cloned in Escherichia coli"

NUCLEIC ACIDS RESEARCH, vol. 10, no. 7, 1982, pages 2177-2187, IRL Press Ltd., London (GB); IRL Press Ltd., London (GB), T.J.R. HARRIS et al.: "Molecular cloning and nucleotide sequence of cDNA coding for calf preprochymosin"

NATURE, vol. 281, 18th October 1979, pages 544-548, MacMillan Journals Ltd.; D.V. GOEDDEL et al.: "Direct expression in Escherichia coli of a DNA sequence coding for human growth hormone"

**Description**

The present invention relates to recombinant DNA and plasmids comprising specific structural genes of mammalian origin coding for the various allelic and maturation forms of preprochymosin, particularly those of bovine origin, and the use of said recombinant plasmids to transform microorganisms in which said genes are expressed.

Chymosin is a protein originating from the stomach of newborn mammals. The bovine type (EC 3.4.23.4) is secreted as an inactive precursor, prochymosin, which consists of a single polypeptide chain of 365 amino acid residues (B. Foltmann *et al.*; Proc. Natl. Acad. Sci. USA; *74* 2321—2324, 1977; B. Foltmann *et al.*; J. Biol. Chemistry; *254*, 8447—8456, 1979). Applicants have found that the precursor of bovine prochymosin, preprochymosin, consists of a single peptide chain of 381 amino acid residues and differs from prochymosin in an amino-terminal of 16 amino acids. This extension very much resembles a signal sequence which is involved in the process of cotranslational excretion (G. Blobel & D. Dobberstein, J. Cell. Biol. *67*, 835—851, 1975).

Prochymosin is irreversibly converted into active enzyme (chymosin) by limited proteolysis, during which a total of 42 amino acid residues are released from the amino-terminal part of the peptide chain. The activation is effected through a pH-dependent two-step autocatalytic conversion. The intermediate, pseudochymosin, is formed by proteolytic cleavage of bond 27—28 at pH 2—3. The final product, chymosin, is formed by activation of pseudochymosin at pH 4—5, (V. Barkholt Pattersen *et al.*, Eur. J. Biochem., *94*, 573—580, 1979).

The enzymatic activity of chymosin consists of the specific proteolysis of K-casein. This property makes chymosin widely used as milk-clotting enzyme in cheese manufacture.

Chymosin is the essential milk-clotting component of rennet, the crude extract of the abomasum of bovine calves. Rennet is used in the production of several types of cheeses in almost all parts of the world. Because of the ever increasing shortage of calf rennet created by the incresaing demand for cheese, many laboratories have been searching for substitutes of microbial origin. Many microbial proteases have been screened, only a few could be used in cheese making, and even these substitutes exhibit a different specificity and therefore may cause an unacceptable texture and/or bitter taste of the cheese. So the production of chymosin by recombinant DNA-containing microorganisms is expected to become of great economical importance.

Developments in recombinant DNA technology have made it possible to isolate or synthesize specific genes or portions thereof from higher organisms, such as man and other mammals, and to transfer these genes or fragments to microorganisms such as bacteria or yeasts. The transferred gene is replicated and propagated as the transformed microorganism replicates. As a result, the transformed microorganism may become endowed with the capacity to make whatever protein the gene or gene fragment encodes, whether it is an enzyme, a hormone, an antigen, an antibody, or a portion thereof. The microorganism passes on this capability to its progeny, so that in effect, the transfer has resulted in a new microbial strain, having the described capability. See, for example, Ullrich, A. *et al.*, Science *196*, 1313 (1977), and Seeburg, P. H. *et al.*, Nature *270*, 486 (1977). A basic fact underlying the application of this technology for practical purposes is that DNA of all living organisms, from microbes to man, is chemically similar, being composed of the same four nucleotides. The significant differences lie in the sequences of these nucleotides in the polymeric DNA molecule. The nucleotide sequences are mainly used to specify the amino acid sequences of proteins that comprise the organism. Although most of the proteins of different organisms differ from each other. The coding relationship between nucleotide sequence and amino acid sequence is fundamentally the same for all organisms.

For example, the same nucleotide sequence which codes for the amino acid sequence of HGH in human pituitary cells, will, when transferred to a microorganism, be recognized as coding for the same amino acid sequence.

For economic reasons it is important that proteins encoded by the recombinant DNA gene are produced under optimal conditions in approved edible micro-organisms as host cells. The main routes to achieve this are:

(1) integration of the structural gene downstream of an effective regulon, in such a way that under selected growth conditions, the amount of protein produced per cell (by an optimal number of cells) is as high as possible.

For that purpose regulons like the double lac UV5, the trp regulon, the double trp regulon of *E. coli* and the regulon of the gene VIII product of the bacteriophages M13, fd and fl are, amongst others, adequate in their natural state or in their modified form(s).

Another factor influencing the yield of required protein per cell is the increase (amplification) of the copy number of the plasmids containing the above-described regulons and the structural genes. Amplification can be effected by the use of a thermosensitive replication mutant derived from the cloacin DF 13 plasmid (pVU 208).

(2) excretion of said protein by microbial host cells into their periplasmic space and/or into the culturing medium, thus preventing said protein from intracellular degradation or preventing the disturbance of normal cellular processes due to too high an intracellular level of said protein. It is now

generally accepted that in many prokaryotic and eukaryotic cells a special $NH_2$-terminal amino acid sequence of the unprocessed form of the proteins is involved in the protein excretion process.

G. Blobel & B. Dobberstein (1975), J. Cell Biol. *67*, 835—851.

In the present invention use is made of recombinant DNA and other molecular biological techniques to construct recombinant DNA molecules that fulfil the above-described requirements. The present invention is also related to the change of the genetic information of structural genes using site-directed mutagenesis.

For a better understanding of the invention the most important terms used in the description will be defined:

An operon is a gene comprising a particular DNA sequence (structural) gene(s) (for polypeptide(s) expression) and a control region or regulon (regulating said expression) and mostly consisting of a promotor sequence, an operator sequence, a ribosome binding- or interaction DNA sequence.

Structural genes are DNA sequences which encode through a template (mRNA) a sequence of amino acids characteristic for a specific polypeptide.

A promoter is a DNA sequence within the regulon to which RNA polymerase binds for the initiation of the transcription.

An operator is a DNA sequence within the regulon to which a repressor protein may bind, thus preventing RNA polymerase from binding to the adjacent promoter.

An inducer is a substance which deactivates a repressor protein, freeing the operator and permitting DNA polymerase to bind to the promoter and start transcription.

Cloning vehicle. A non-chromosomal double-stranded DNA, plasmid or phage, comprising a DNA sequence (intact replicon) that allows self-replication after transformation into suitable host cells.

Phage or bacteriophage. Bacterial virus which can replicate in a suitable bacterial host cell.

Reading frame. The grouping of triplets of nucleotides (codons) into such a frame that at mRNA level a proper translation of the codons into the polypeptide takes place.

Transcription. The process of producing RNA from a structural gene.

Translation. The process of producing a polypeptide from mRNA.

Expression. The process undergone by a structural gene to produce a polypeptide. It is a combination of many processes, including at least transcription and translation.

Thermosensitive replication mutant. A plasmid containing a mutation in its replication origin which causes its copy number to be temperature dependant.

Allelic form. One of two or more naturally occurring alternative forms of a gene product.

Maturation form. One of two or more naturally occurring forms of a gene product procured by specific processing, e.g. specific proteolysis.

Plus strand. DNA strand whose nucleotide sequence is identical with the mRNA sequence, with the proviso that uracil is replaced by thymidine.

By maturation forms of preprochymosin are meant prochymosin, pseudochymosin and chymosin.

Prochymosin arises through the action of signal peptidase on preprochymosin, which causes the loss of the amino terminal—excretion related—signal sequence.

The chemical structure of bovine preprochymosin is given in Fig. 1. Bovine prochymosin corresponds with residues 1—365 (Fig. 1). Pseudochymosin arises through the autocatalytic proteolysis of prochymosin at pH 2, causing the loss of an amino terminal portion of prochymosin. The chemical structure of bovine pseudochymosin corresponds with residues 28—365 of preprochymosin given in Fig. 1.

Chymosin arises through the autocatalytic proteolysis of pseudochymosin at pH 4—5, causing the loss of an amino terminal portion of pseudochymosin. The chemical structure of bovine chymosin corresponds with residues 43—365 of preprochymosin given in Fig. 1.

According to the invention there is provided structural gene(s) coding for the various allelic and maturation forms of mammalian *preprochymosin*, particularly bovine preprochymosin according to Fig. 1 and 2; and further a recombinant plasmid comprising:

(i) structural gene(s) coding for the various allelic and maturation forms of mammalian *preprochymosin*, particularly bovine preprochymosin according to Fig. 1 and 2;

(ii) specific DNA sequences which regulate the expression of said structural genes in a microbial host.

These specific DNA sequences consist of either an inducible or a constitutive regulon. A preferred inducible regulon consists of a double lac UV5 system as described by D. V. Goeddel *et al.*, Nature *281*, 544—548 (1978). (See Fig. 8).

Another preferred inducible regulon is a constituent of the tryptophan system described by F. Lee *et al.*, J. Mol. Biol. *121*, 193—217 (1978) and K. Bertrand *et al.*, Science *189*, 22—26 (1975). Applicants have modified this tryptophan system to obtain a more adequate system according to Fig. 9. In this modified system the information coding for the trp attenuator protein is eliminated while maintaining its riposome-binding site. Expression is highly increased when two *trp* regulons in a head to tail fashion are being used. Synthesis of this system is illustrated in Fig. 11.

The recombinant plasmid according to the invention may comprise DNA sequences which regulate the expression of the structural genes, preferably consisting of a modified promoter/ribosome-binding site of gene VIII of bacteriophage M13, fd or fl (P.M.G.F. van Wezenbeek *et al.*, Gene *11*, 129—148 (1980)).

Efficiency of required protein per cell was highly increased when in conjunction with the regulon systems described above, the copy number of the recombinant cloning vehicle was increased. This was

effected by the use of a thermosensitive replication mutant derived from the cloacin DF13 plasmid pVU 208 (A. Stuitje, thesis, V. U. Amsterdam, 1981). Increase in temperature results in a ten- to hundred-fold increase in copy number. The construction of such plasmids is illustrated in Fig. 12 and 13.

In the recombinant plasmid according to the invention the regulon may be either directly linked to the structural gene or indirectly through a novel start codon and EcoRI-site containing DNA linker comprising the nucleotide sequence

$$5'pCAT(N)_nGAATTC(N')_nATG(3')_{OH}$$

wherein n=0, 1, 2 or 3 and

N and N' are any of the nucleotides A, T, G or C, with the proviso that in the double-stranded structure N and N' are such that a rotational symmetrical structure is present.

By a rotational symmetrical structure is meant that where N is e.g. represented by A, N' should be represented by the complementary base T. In some instances it turned out that the yield of expression improved when the sequence AATT between the regulon and the structural gene has been eliminated.

Allelic forms of bovine preprochymosin have been constructed departing from the nucleotide sequence given in Fig. 1. Examples of these constructions are outlined in Fig. 18 and construction step 10e described further in the specification, whereby use is made of site-directed mutagenesis. The latter procedure could also be applied usefully so as to produce preprochymosin with a specifically altered signal sequence which allows efficient excretion in microbial hosts and prochymosin with an improved acid-induced autocatalytic proteolysis characteristic.

The microbial cloning vehicles containing the structural genes encoding the various allelic and maturation forms of preprochymosin according to the invention are produced by a number of steps, the most essential of which are:

1. Isolation and enrichment of the messenger RNA(mRNA) of preprochymosin.
2. Conversion of this mRNA into double stranded DNA (dsDNA).
3. Construction of dsDNA having a poly-dC-tail.
4. Incorporation of the dsDNA-poly-dC molecules in PstI-cleaved and poly-dG-tailed pBR 322 DNA.
5. Transformation of competent *E. coli* cells and selection of tetracycline resistant colonies.
6. Determination of the nature of the inserts by RNA/DNA hybridization and *in vitro* translation; and by DNA/DNA hybridization using a specific $^{32}P$-labelled cDNA probe.
7. Double checking the nature of the cloned PstI-inserts by DNA- and RNA-sequence analysis.

8a. Producing DNA encoding the amino terminal part of pseudochymosin plus a translational initiation ATG-codon (added) at the amino terminus.

8b. Producing DNA encoding the amino terminal part of chymosin plus a translational initiation ATG-codon at the amino terminus.

8c. Producing DNA encoding the amino terminal part of prochymosin plus a translational initiation ATG-codon at the amino terminus.

8d. Producing DNA encoding the amino terminal part of preprochymosin plus a translational initiation ATG-codon at the amino terminus.

9. Construction of plasmids comprising a constitutive or inducible regulon, with or without a thermosensitive replication mutation.
10. Construction of plasmids consisting of plasmids described under 9 and the ligated preprochymosin gene or its various maturation forms, and transformation of said plasmids into microbial host cells.
11. Culturing of microbial cells, e.g. *E. coli* cells, containing recombinant plasmids described under 10 and detection and isolation of preprochymosin, prochymosin, pseudochymosin or chymosin therefrom. Culturing conditions were optimized as to the yield of preprochymosin or its maturation forms per cell. Conversion into enzymatically active chymosin of the various precursors was also optimized.

The following description will illustrate the above-mentioned steps in detail.

1. Isolation and purification of bovine (prepro)chymosin mRNA

The fourth stomach of a preruminant calf (abomasum, Frisian cow) was ground under liquid nitrogen, extracted with phenol and a selective precipitation of the RNAs with LiCl was performed following the procedure described by K. S. Kirby, Biochem, J. *96*, 266—269 (1965), U. Wiegers & H. Hilz (FEBS. Letters, *23*, 77—82 (1972). PolyA-containing mRNA was recovered by several passages over oligo-dT-cellulose columns as described by H. Aviv & P. Leder (Proc. Natl. Acad. Sci. USA, *69*, 1408—1412 (1972)).

2. Conversion of (prepro)chymosin mRNA into double-stranded DNA

Purified (prepro)chymosin mRNA was copied with AMV reverse transcriptase to yield a single-stranded DNA molecule, according to the procedure described by G. N. Buell *et al.*, J. Biol. Chem. *253*, 2471—2482 (1978). This cDNA was subsequently converted into a double-stranded molecule using DNA-polymerase, according to the procedure described by A. R. Davis *et al.*, Gene *10*, 205—218 (1980). Thereafter the loop structure of the double-stranded DNA copy was removed by S1-nuclease digestion.

3. Construction of double-stranded DNA with poly-dC-tails

DNA molecules of the desired length were obtained by polyacrylamide gel-electrophoresis, extracted from the gel and tailed with poly-dC by terminal transferase according to the procedure described by R. Roychoudhury *et al.*, Nucleic Acids Research *3*, 863—877 (1976).

4. Integration of the dsDNA-poly-dC molecules in plasmid pBR 322

Plasmid pBN 322 was treated with restriction endonuclease PstI, that cleaves the plasmid at a recognition site that lies in the gene encoding the ampicillin resistance, whereafter the linearized DNA of pBR 322 was supplied at the PstI-site with poly-dG-tails by terminal transferase. The poly-dC-tailed DNA molecules were annealed to the poly-dG-tailed plasmid pBR 322.

5. Transformation and clone selection

The plasmids thus obtained were transferred into $CaCl_2$-treated *E. coli* cells. After transformation, cells containing hybrid plasmid DNA molecules were selected on their resistance to tetracycline. (M. Mandel & A. Higa, J. Mol. Biol., *53*, 159—162 (1970).

6. Determination of the nature of the inserts (I) DNA/DNA colony hybridization and RNA/DNA hybridization/*in vitro* translation

Positive colonies were further selected by colony hybridization (R. E. Thayer, Anal. Biochem., *98*, 60—63 (1979)) with a radioactively labelled, chymosin-specific cDNA. The latter product was obtained by priming reverse transcription (see 2) of the mRNA preparation (see 1) with the oligonucleotide (5') $dTTCATCATGTT_{OH}$(3'). This oligonucleotide was designed by the Applicants, because it represents one of the two theoretically possible nucleotide stretches coding for the unique amino acid sequence -asN-met-met-asN-, which occurs in the (prepro)chymosin molecule at position 183—186 (Fig. 1). Upon priming cDNA synthesis with this undecanucleotide a distinct cDNA product with a chain length of circa 650 nucleotides was obtained. This product was isolated and used as a probe in the colony hybridization experiments. Several positive colonies could be identified and, as a double check on the identity of the cloned DNA, the plasmid DNA from some of these colonies was isolated and used in the hybridization/*in vitro* translation procedure described by J. G. Williams *et al.* (Cell, *17*, 903—913 (1979)).

7. Determination of the nature of the inserts (II) by DNA/RNA sequence analysis (Fig. 1)

The nucleotide sequence analysis of the (prepro)chymosin inserts was performed by the chemical degradation procedure as outlined by A. M. Maxam & W. Gilbert in Methods in Enzymology, L. Grossman & K. Moldave editors, New York, Acad. Press, 1980, Vol. 65 (1), pages 499—560, and the dideoxy/nick translation procedure as outlined by J. Maat & A. J. H. Smith, Nucleic Acid Research, *5*, 4537—4545 (1978). Further information on the nucleotide sequence of the (prepro)chymosin mRNA was derived indirectly by primed synthesis by AMV-reverse transcriptase on the (prepro)chymosin mRNA template in the presence of chain-terminating inhibitors, as outlined by D. Zimmern & P. Kaesberg, Proc. Natl. Acad. Sci. U.S.A. *75*, 4257—4261 (1978). This screening yielded *inter alia* plasmid pUR 1001 containing an almost complete copy of preprochymosin mRNA.

8a. Production of DNA encoding the amino terminal part of pseudochymosin ATG (Fig. 3, 4)

Numbers refer to the preprochymosin mRNA sequence in Fig. 1, unless indicated otherwise.

Plasmid pBR 322 was cleaved with the restriction enzyme HaeIII and the resulting fragments were subsequently blunt-end ligated with synthetic HindIII-linkers (5') dCCAAGCTTGG (3'). The mixture was subsequently incubated with HindIII and phosphatase. The reactions were terminated by protein extraction with phenol/chloroform (50/50 v/v) and the DNA was then cleaved with PstI. The resulting mixture was subjected to polyacrylamide gel electrophoresis and a 148 bp fragment (fragment A, Fig. 3) extending from position 3608—3756 in the pBR 322 DNA sequence. (J. G. Sutcliffe, Cold Spring Harbor Symposia on Quantitative Biology, *43*, 77—90 (1978)) was isolated from the gel by electroelution.

Plasmid pUR 1001 was cleaved with EcoRI and treated with calf phosphatase. The mixture was extracted with phenol/chloroform and subsequently PstI was added. Resulting fragments were separated by agarose electrophoresis. Fragment B (see Fig. 3) extending from the EcoRI-site at position 549 to the PstI-site in the noncoding sequence of the preprochymosin gene at the carboxy terminal and of the pUR 1001 clone, was isolated.

Fragments A and B were ligated with the large EcoRI-HindIII fragment of pUR 201, 301, 401, 303, 210, 310, 410, 311 (combined called fragment C) yielding pUR 1520, 1530, 1540, 1730, 1820, 1830, 1840, 1930, respectively.

Subsequently plasmid pUR 1001 DNA was cleaved with PstI and resulting DNA fragments were ligated to the synthetic pentanucleotide (5') $d_{HO}$CTGCA (3'). Following ligation, the mixture was incubated with *E. coli* DNA polymerase, large fragment, in the presence of dGTP in order to make blunt ends. The DNA was phosphorylated using $T_4$ kinase and ATP, and subsequently supplied with synthetic EcoRI-linkers of the structure (5')dCAT$(N)_n$GAATTC$(N')_n$ATG(3'), wherein n=0, 1, 2 and 3 and N and N' are any of the deoxynucleotides A, C, G or T, with the proviso that in the double-stranded structure N and N' are such that

a rotational symmetric structure is present. The DNA was then treated with EcoRI and resulted in fragment (I) of circa 400 bp in length, which was isolated (Fig. 4).

8b. Production of DNA encoding the amino terminal part of chymosin (Fig. 5)

Plamsid pUR 1001 was cleaved with PstI and the 1300 bp PstI insert was isolated. This DNA fragment was heat-denaturated to produce single strands. The synthetic primer (5') dGGGGAGGTGG(3') was used to produce complementary DNA synthesis starting from position 198 in the direction of the carboxy terminus by the action of EcoRI DNA polymerase, large fragment. Subsequently the DNA was treated with nuclease S1 to procure blunt-ended dsDNA. To this dsDNA the synthetic EcoRI-linker (5') dCAT$(N)_n$GAATTC$(N')_n$ATG (3') was ligated. After digestion with EcoRI and treatment with phosphatase, the DNA was cleaved once more by BglII. The resulting fragment II (Fig. 5) was isolated.

8c. Production of DNA encoding the amino terminal part of prochymosin (Fig. 6)

Plasmid pUR 1001 was treated with HphI, followed by nuclease S1. A 202 base pair long fragment III was isolated (Fig. 6). Fragment III was then ligated to the synthetic EcoRI-linker (5')dCAT$(N)_n$GAATTC$(N')_n$ATG, cleaved with EcoRI and dephosphorylated with phosphatase. The resulting DNA was digested once more by BglII and the resulting fragment IV was isolated.

8d. Production of DNA encoding the amino terminal part of preprochymosin (Fig. 7)

Plasmid pUR 1001 was cleaved with PstI and EcoRI. A fragment of 396 bp was isolated. This fragment was then treated with exonuclease III to procure single-stranded non-complementary DNA (A. J. H. Smith (1979), Nucleic Acids Res. *6*, 831—841).

This DNA was hybridized to preprochymosin mRNA under conditions described by G. Akusjärvi and U. Petterson (1978), Proc. Natl. Acad. Sci. U.S.A., *75*, 5822—5826. The cDNA synthesis was performed as described under 2. Following heat denaturation, dsDNA was made using DNA polymerase, large fragment, with (5')dAGGTGTCTC$G_{OH}$(3') acting as a primer. The dsDNA was treated with nuclease S1 and ligated to the synthetic EcoRI-linker dCAT$(N)_n$GAATTC$(N')_n$ATG. After EcoRI cleavage and dephosphorylation wth (calf intestinal) phosphatase, the DNA was split once more with BglII. The resulting circa 230 bp long fragment (V) was isolated.

9. Construction of plasmids comprising a constitutive or inducible regulon, with or without a thermosensitive replication mutation

9a. Construction of a plasmid pUR 201 (Fig. 8)

A fragment containing 285 base pairs comprising double lac regulon (lac UV5) was obtained by restriction endonuclease EcoRI cleavage of pKB 268, described by K. Backman & M. Ptashne, Cell *13*, 65—71 (1978). This fragment was ligated in the EcoRI-site of pBR 322 DNA. Plasmid DNA with the lac regulon in the right orientation, pUR 200, (Fig. 8) was partly cleaved by EcoRI in the presence of *E. coli* RNA polymerase. The EcoRI cleavage site most distant from the restriction endonuclease HindIII cleavage site was preferentially attacked. The linearized DNA was treated with S1 nuclease, purified by agarose gel electrophoresis, circularized by ligation with T4 DNA-ligase and subsequently transformed into *E. coli*. From the tetracycline-resistant transformants pUR 201 with the correct structure (Fig. 8) was obtained.

9b. Construction of plasmid pUR 301 (Fig. 9)

A DNA fragment of about 510 base pairs containing the trp regulon was obtained by restriction endonuclease HinfI cleavage of ptrp ED5, as described by R. A. Hallewell & S. Emtage, Gene *9*, 27—47 (1980). This fragment was cleaved with restriction endonuclease TaqI in the presence of *E. coli* RNA polymerase. The TaqI-site in the trp regulon (described by K. Bertrand *et al.*, Science 189, 22—26 (1975) and F. Lee *et al.*, J. Mol. Biol. *121*, 193—217 (1978)) was selectively protected, thus yielding a fragment containing 234 base pairs comprising the trp regulon (Fig. 9). This fragment was then treated with S1 nuclease, blunt-end ligated with the EcoRI-linker (5') dGGAATTC$C_{OH}$(3'), cut with EcoRI and subsequently cloned in the EcoRI-site of pBR 322.

Plasmid pUR 300 with the trp regulon in the correct orientation (Fig. 9) was isolated. The EcoRI-cleavage site most distant from the HindIII-site was removed by partial cleavage of pUR 300 DNA by EcoRI in the presence of ethidium bromide and S1 nuclease treatment. Linear DNA molecules were recircularized by T4 DNA ligase. From the tetracycline-resistant transformants pUR 301 with the structure as outlined in Fig. 9 was obtained.

9c. Construction of plasmid pUR 401 (Fig. 10)

A 270 base pairs fragment comprising the gene VIII-promotor was obtained by digestion of RF M13 DNA (DNA sequence 1128—1397 see P.M.G.F. van Wezenbeek *et al.*, Gene *11*, 129—148 (1980), with the restriction nucleases TaqI and HaeIII; the TaqI-site was made blunt-ended by a repair reaction with *E. coli* DNA polymerase; the fragment was subsequently partly digested with restriction enzyme MnlI. The partial products were treated with T4 DNA polymerase and S1 nuclease and subsequently blunt-end ligated with the EcoRI-linker (5')dGGAATTC$C_{OH}$(3'), then treated with EcoRI and ligated in the EcoRI-site of the pBR 322.

By restriction enzyme analysis and DNA sequencing, a plasmid was isolated in which the EcoRI-cleavage site was located just beyond the ribosome-binding site of the M13 gene VIII DNA sequence. Applicants have found that the plasmids having the M13 regulon from nucleotide 1128 to nucleotides 1291 to 1297 were appropriate regulons for expression. The EcoRI-cleavage site most distant from the HindIII-site was removed essentially as described for pUR 301. The complete construction of pUR 401 is outlined in Fig. 10.

9d. Construction of plasmid pUR 303 (Fig. 11)

Plasmid pUR 300 (9b, Fig. 9) was digested with EcoRI and the 234 bp fragment comprising the trp regulon was isolated. This fragment was ligated to pUR 301 DNA, which previously had been cleaved with EcoRI and dephosphorylated with phosphatase. The ligation mixture was used to transform competent *E. coli* cells and from the ampicillin-resistant transformants pUR 302 was obtained. This plasmid comprises two trp regulons with identical transcription polarity (Fig. 11). pUR 302 was partially cleaved with EcoRI, in the presence of ethidium bromide, treated with nuclease S1 to generate blunt-ends and the cleaved plasmid DNA's were religated. The ligation mix was used to transform competent *E. coli* cells and from the ampicillin-resistant transformants pUR 303 was isolated, wherein the EcoRI-site in between the two trp regulons in pUR 302 had been removed.

9e. Construction of pUR 10 (Fig. 12)

Plasmid pBR 322 was cleaved with PstI and PvuII, and subsequently dephosphorylated with phosphatase. The 2817 bp long fragment (D, Fig. 12) was isolated.

Plasmid pBR 322 was also cleaved with MboII, treated with nuclease S1 and phosphatase and then cleaved once more with PstI. The 400 bp long fragment (E. Fig. 12) extending from position 3201—3608 (J. G. Sutcliffe, Cold Spring Harbor Symposia on Quantitative Biology, *43*, 77—90 (1978) was isolated. Plasmid pVU 208 (A. R. Stuitje, thesis, V. U. Amsterdam (1981) was cleaved with BamHI and treated with nuclease S1. The 760 bp fragment (F, Fig. 12) containing the replication origin of clo DF 13 with the cop ts mutation, was isolated.

To construct pUR 10, fragments D and E were ligated first, followed by ligation with fragment F. The ligation-mix was used to transform competent *E. coli* cells. From ampicillin- and tetracycline-resistant transformants pUR 10-containing cells were isolated. The replication-origin-containing fragment is oriented such that the unidirectional replication is in a counter-clockwise direction.

9f. Construction of pUR 210, pUR 310, pUR 311, pUR 410 (Fig. 13)

These plasmids are derived from pUR 201, pUR 301, pUR 303 and pUR 401, respectively, and contain the cop ts replication origin of pUR 10. pUR 10 was digested with PstI and BamHI and the 2841 bp long fragment (G, Fig. 13) was isolated by agarose gel electrophoresis and electroelution.

Each of the plasmids pUR 201, pUR 301, pUR 303 and pUR 401 was digested with PstI and BamHI and the "regulon"-containing fragments (collectively called H, Fig. 13) were isolated. Fragment G and each of the fragments H in turn were ligated using T4 DNA ligase and the ligation mixes were used to transform competent *E. coli* cells. From ampicillin- and tetracycline-resistant colonies pUR 210-, pUR 310-, pUR 311- and pUR 410-containing cells were isolated.

10. Construction of plasmids comprising a constitutive or inducible regulon and the ligated preprochymosin gene or its various allelic and maturation forms, the latter being under transcriptional control of said regulons, and transformation of said plasmids into *E. coli*

10a. Construction of expression plasmids giving rise to the synthesis of bovine pseudochymosin (Fig. 14)

Plasmids pUR 1520, 1530, 1540, 1730, 1820, 1830, 1840 and 1930 were cleaved with EcoRI and dephosphorylated. Each preparation in turn was ligated with fragment I (8a, Fig. 4). This ligation mix was used to transform competent *E. coli* RRI and from the ampicillin resistant transformants, cells containing pUR 1521, 1531, 1541, 1731, 1821, 1831, 1841 and 1931 were selected which contained fragment I inserted such that the genetic information coding for pseudochymosin was present as a continuous uninterrupted entity.

10b. Construction of expression plasmids giving rise to the synthesis of chymosin (Fig. 15)

Plasmid pUR 1521 was cleaved with HindIII, dephosphorylated and then cleaved once more with BglII. The resulting ~1300 bp long fragment VI (Fig. 15) was purified. The vector fragments C (8a, Fig. 3), in turn, were ligated with fragments II (8b, Fig. 5) and fragment VI. The ligation mix was used to transform competent *E. coli* cells and from ampicillin-resistant transformants cells containing pUR 1522, 1532, 1542, 1732, 1822, 1832, 1842 and 1932 were selected.

10c. Construction of expression plasmids giving rise to the synthesis of prochymosin (Fig. 16)

Vector fragments C (8a, Fig. 3), fragment IV (8c, Fig. 6) and fragment VI (10b Fig. 15) were ligated and the resulting ligation mix was used to transform competent *E. coli* cells. From the ampicillin-resistant transformant cells containing pUR 1523, 1533, 1543, 1733, 1823, 1833, 1843 and 1933 were selected.

10d. Construction of expression plasmids giving rise to the expression of preprochymosin (Fig. 17)

Vector fragments C (8a, Fig. 3), fragment V (8d, Fig. 7) and fragment VI (9b, Fig. 15) were ligated and the resulting ligation mix was used to transform competent *E. coli* cells.

From the ampicillin-resistant transformants cells containing pUR 1524, 1534, 1544, 1734, 1824, 1834, 1844 and 1934 were selected (Fig. 17).

10e. Example demonstrating the use of site-directed mutagenesis to create allelic forms of the bovine preprochymosin or its maturation forms, departing from the chemical structure given in Fig. 1 and thereby converting residues 202 and 286 into aspartic acid residues, such in turn or in combination (Fig. 18—22)

Plasmid pUR 1001 was cleaved with PstI and the resulting DNA fragments were ligated to the synthetic pentanucleotide $(5')_{HO}dCTGCA_{OH}(3')$. Following ligation, the mixture was incubated with *E. coli* DNA polymerase, large fragment in the presence of dGTP in order to make blunt-ends and phosphorylated with T4 kinase and ATP. The DNA was subsequently supplied with EcoRI-linker (5')dCATGAATTCATG(3') and then treated with EcoRI. A circa 880 bp long fragment extending from the EcoRI-site at position 549 to the carboxy terminal end of the chymosin encoding DNA was isolated. This fragment was subsequently cloned in the EcoRI-site of RFM13 mp 2. Two clones were isolated, M13 1020 and M13 1021, which were different in the orientation of the EcoRI-insert with respect to each other (cf. Fig. 18). M13 1020 contained the coding strand (plus strand); M12 1021 contained the non-coding strand (minus strand). ss.Phage DNA of M13 1020 was converted into double-stranded DNA using *E. coli* DNA polymerase large fragment, dNTP's and

(5')dTGGCCATCCCTGTCC(3') i

or

(675)

(5')dAAACTCATCGTACTG(3') ii

(928)

in turn as primers, using procedures described by S. Gillam *et al.* (1979); Nucleic Acids Res., *6*, 2973—2985.

The underlined bases represent mismatches in the primer/template hybrid. Following transformation of competent *E. coli* JM101.7118 cells (B. Gronenborn & J. Messing, Nature, *272*, 375—377 (1978)), phages were screened for the required conversion into the chymosin encoding sequence by plaque hybridization with the $^{32}p$ labelled pentadecanucleotides i, ii, as probes and DNA-sequence analysis.

Two phage isolates, M13.1022 and 1023 contained the DNA-sequences:

```
       asp  arg  asp  gly
(5')—GGAC  AGG  GAT  GGC  CA—(3')      M13.1022

                675

      glN  tyr  asp  glu  phe
(5')—CAG  TAC  GAT  GAG  TTT—(3')      M13.1023

                928
```

RF M13.1022 and FR M13.1023 were cleaved with EcoRI, dephosphorylated and then cleaved with PstI. From each preparation an 888 bp long fragment was isolated by agarose gel electrophoresis and electroelution. Apart from the required mutations, this fragment corresponds with fragment B (8a, Fig. 3).

Using procedures identical with those described in 8a—8c, expression plasmids were constructed which gave rise to the synthesis of specifically altered pseudochymosin, chymosin, prochymosin and preprochymosin, respectively.

11. Culturing of *E. coli* cells containing recombinant plasmids described under 10 and detection and isolation of preprochymosin, prochymosin, pseudochymosin or chymosin

*E. coli* cells containing one of the plasmids pUR 1521 (ATCC 39120), 1531, 1541, 1731, 1821, 1831, 1841, 1931 pUR 1522, 1532, 1542, 1732, 1822, 1832 (ATCC 39197), 1842, 1932 pUR 1523, 1533 (ATCC 39121), 1543, 1733, 1823, 1833, 1843, 1933 pUR 1524, 1534, 1544, 1734 (ATCC 39198), 1824, 1834, 1844, 1934 with or without the AATT-sequence in the linker between the regulon and the preprochymosin genes or its maturation forms were cultured under optimal conditions for their growth. These culturing conditions vary with the type of plasmid present in the cells, but a suitable antibiotic (ampicillin) was always present to maintain selection pressure.

Under these conditions the cells containing either plasmids pUR 1521, 1531, 1541, 1731, 1821, 1831, 1841, 1931 or pUR 1522, 1532, 1542, 1732, 1822, 1832, 1842, 1932 or pUR 1523, 1533, 1543, 1733, 1823, 1833, 1843, 1933 or pUR 1524, 1534, 1544, 1734, 1824, 1834, 1844, 1934 produced considerable amounts of

pseudochymosin, chymosin, prochymosin or preprochymosin, respectively. These amounts varied from $10^3$—$10^7$ molecules/cell.

*E. coli* cells which contained preprochymosin or modified preprochymosin encoding plasmids contained (modified) preprochymosin in the cytoplasm and prochymosin in their periplasmic space.

The bacterially produced preprochymosin methionyl, prochymosin and methionyl pseudochymosin could be converted into chymosin using the procedures described by V. Barkholt Pedersen *et al.* (Eur. J. Biochem., *94,* 573—580 (1979)). The chymosins which were thus obtained and bacterially-produced chymosin were shown to be fully biologically active in proteolysis.

The presence of the proteins was further demonstrated by SDS-polyacrylamide gel electrophoresis with or without immunoprecipitation, and by immunological ELISA and RIA tests. The antisera for this test were generated by injecting bovine calf chymosin supplemented with Freund adjuvant into sheep as well as rabbits.

The above description was focussed on the synthesis of chymosin in *E. coli* cells; it is of course possible and desirable to use for that purpose non-toxic, edible micro-organisms, such as streptococci or micro-organisms of Bacillus or yeast origin.

Abbreviations

| | |
|---|---|
| DNA | deoxyribonucleic acid |
| cDNA | complementary DNA |
| dsDNA | double-stranded DNA |
| RNA | ribonucleic acid |
| mRNA | messenger RNA |
| A | adenine |
| G | guanine |
| C | cytosine |
| T | thymine |
| RBS | ribosome-binding site |
| N | any nucleotide |
| bp | base pair |
| M13 | bacteriophage M13 |
| RF | replicative form |
| p | plasmid |
| $^{32}$p | phosphorus 32 |
| n | number |
| p/o | promotor/operator |
| trp | tryptophan |
| lac | lactose |
| EcoRI | restriction endonuclease derived from *Escherichia coli* RYI |
| HindIII | restriction endonuclease derived from *Haemophilus influenzae* RdIII |
| Hinfl | restriction endonuclease derived from *Haemophilus Influenzae* |
| HaeIII | restriction endonuclease derived from *Haemophilus aegypticus* |
| BglII | restriction endonuclease derived from *Bacillus globii* |
| Hphl | restriction endonuclease derived from *Haemophilus parahaemolyticus* |
| PstI | restriction endonuclease derived from *Providencia stuartii* |
| MnII | restriction endonuclease derived from *Moraxella nonliquefaciens* |
| TaqI | restriction endonuclease derived from *Thermophilus aquaticus* |
| BamHI | restriction endonuclease derived from *Bacillus amyloliquefaciens* H |
| *E. coli* | *Escherichia coli* |
| ts | thermosensitive |
| DdeI | restriction endonuclease derived from *Desulfovibrio desulfuricans* |
| HpaII | restriction endonuclease derived from *Haemophilus parainfluenzae* |
| ELISA | enzyme linked immuno sorbent assay |
| RIA | radioimmune assay |
| SDS | sodium dodecyl sulphate |
| met | methionine |
| leu | leucine |
| ile | isoleucine |
| ala | alanine |
| asp | aspartic acid |
| asN | asparagine |
| glu | glutamic acid |
| glN | glutamine |
| val | valine |
| thr | threonine |
| phe | phenylalanine |

Abbreviations (continued)

| | |
|---|---|
| tyr | tyrosine |
| cys | cysteine |
| arg | arginine |
| ser | serine |
| his | histidine |
| pro | proline |
| gly | glycine |
| lys | lysine |
| Ap | ampicillin resistance |
| Tc | tetracycline resistance |

Legends to the figures:

Fig. 1. Amino acid sequence of one of the allelic forms (B) of bovine preprochymosin the DNA sequence corresponding to the bovine preprochymosin B mRNA.

The S numbering denotes numbering of the signal sequence amino acid residues.

Fig. 2. Schematic representation of several examples of allelic forms of bovine preprochymosin. The upper drawing corresponds with Fig. 1. The numbering in paretheses denotes the amino acid residues in the preprochymosin molecule. Other numbers refer to the nucleotide sequence of the mRNA.

Fig. 3. Construction route of pUR 1520, 1530, 1540, 1730, 1820, 1840 and 1930, described in 8a.

Fig. 4. Construction route of dsDNA encoding amino terminal end of pseudochymosin plus a transcriptional initiation triplet, described in 8a.

Fig. 5. Construction route of dsDAN encoding the amino terminal end of chymosin plus a transcriptional initiation triplet, described in 8b.

Fig. 6. Construction route of dsDNA encoding the amino terminal end of prochymosin plus a transcriptional initiation triplet, described in 8c.

Fig. 7. Construction route of dsDNA encoding the amino terminal end of preprochymosin, described in 8d.

Fig. 8. Construction route of pUR 201, described in 9a.

Fig. 9. Construction route of pUR 301, described in 9b.

Fig. 10. Construction route of pUR 401, described in 9c.

Fig. 11. Construction route of pUR 303, described in 9d.

Fig. 12. Construction route of pUR 10, described in 9e.

Fig. 13. Construction route of pUR 210, 310, 311 and 410, described in 9f.

Fig. 14. Construction route of pUR 1521, 1531, 1541, 1731, 1821, 1831, 1841 and 1931, described in 10a.

Fig. 15. Construction route of pUR 1522, 1532, 1542, 1732, 1822, 1832, 1842 and 1932, described in 10b.

Fig. 16. Schematic representation of pUR 1523, 1533, 1543, 1733, 1823, 1833, 1843 and 1933, described in 10c.

Fig. 17. Schematic representation of pUR 1524, 1534, 1544, 1734, 1824, 1834, 1844 and 1934, described in 10d.

Fig. 18. Construction route of M13.1020, M13.1021 and part of dsDNA encoding an allelic variation of bovine preprochymosin, described in 10e.

Fig. 19. General representation of the plus strand DNA sequence corresponding to the structural gene encoding preprochymosin wherein:

A is deoxyadenyl,
G is deoxyguanyl,
C is deoxycytoxyl,
T is thymidyl,
J is A or G;
K is T or C;
L is A, T, C, or G;
M is A, C or T;
X is T or C if Y is A or G, and C if Y is C or T;
Y is A, G, C or T if X is C, and A or G if X is T;
W is C or A if Z is G or A, and C if Z is C or T;
Z is A, G, C or T if W is C, and A or G if W is A;
QR is TC if S is A, G, C or T, and AG if S is T or C; and
S is A, G, C or T if QR is TC, and T or C if QR is AG.

Fig. 20. General representation of the plus strand DNA sequence corresponding to the structural gene encoding prochymosin plus a transcriptional initiation ATG-triplet wherein:

A is deoxyadenyl,
G is deoxyguanyl,
C is deoxycytosyl,
T is thymidyl,
J is A or G;

K is T or C;
L is A, T, C, or G;
M is A, C or T;
X is T or C if Y is A or G, and C if Y is C or T;
Y is A, G, C or T if X is C, and A or G if X is T;
W is C or A if Z is G or A, and C if Z is C or T;
Z is A, G, C or T if W is C, and A or G if W is A;
QR is TC if S is A, G, C or T, and AG if S is T or C; and
S is A, G, C or T if QR is TC, and T or C if QR is AG.

Fig. 21. General representation of the plus strand DNA sequence corresponding to the structural gene encoding pseudochymosin plus a transcriptional initiation ATG-triplet wherein:
A is deoxyadenyl,
G is deoxyguanyl,
C is deoxycytosyl,
T is thymidyl,
J is A or G;
K is T or C;
L is A, T, C, or G;
M is A, C or T;
X is T or C if Y is A or G, and C if Y is C or T;
Y is A, G, C or T if X is C, and A or G if X is T;
W is C or A if Z is G or A, and C if Z is C or T;
Z is A, G, C or T if W is C, and A or G if W is A;
QR is TC if S is A, G, C or T, and AG if S is T or C; and
S is A, G, C or T if QR is TC, and T or C if QR is AG.

Fig. 22. General representation of the plus strand DNA sequence corresponding to the structural gene encoding chymosin plus a transcriptional initiation ATG-triplet wherein:
A is deoxyadenyl,
G is deoxyguanyl,
C is deoxycytosyl,
T is thymidyl,
J is A or G;
K is T or C;
L is A, T, C, or G;
M is A, C or T;
X is T or C if Y is A or G, and C if Y is C or T;
Y is A, G, C or T if X is C, and A or G if X is T;
W is C or A if Z is G or A, and C if Z is C or T;
Z is A, G, C or T if W is C, and A or G if W is A;
QR is TC if S is A, G, C or T, and AG if S is T or C; and
S is A, G, C or T if QR is TC, and T or C if QR is AG.

General remarks on the figures

Generally plasmid DNA is drawn as a single-lined circle, still this represents double-stranded DNA (bacteriophage M13.DNA is single-stranded; the replicative form RF, however, is double-stranded). 5′-ends of cleaved DNA at restriction enzyme cleavage site are phosphorylated unless indicated otherwise; 3′-ends are always dephosphorylated.

The numbers given in italics refer to the bovine preprochymosin DNA sequence given in Fig. 1; otherwise they refer to plasmid DNA sequences.

dA/dT-stretch

dG/dC-stretch

regulon, the arrow indicates transcription direction.

**Claims for the Contracting States: BE, CH, DE, FR, GB, IT, LI, NL, SE**

1. A recombinant plasmid comprising the following elements in the order given:
(1) a ds-rDNA coding for preprochymosin, prochymosin, pseudochymosin or chymosin,
(2) a translational stop codon bound to the 3′-end of the plus strand of the ds-rDNA of (1),
(3) both an *E. coli* replication site and a selective marker,
(4) an *E. coli* expression regulon upstream of the plus strand of the ds-rDNA of (1), and
(5) when the ds-rDNA codes for prochymosin, pseudochymosin or chymosin, a translational initiation ATG-triplet bound to the 5′-end of the plus strand of the ds-rDNA of (1).

2. A recombinant plasmid according to Claim 1, wherein the plus strand of the preprochymosin ds-rDNA of (1) is selected from the following sequences
(a) the polynucleotide 24—1166

| | | | | | | | | | | | | |
|---|---|---|---|---|---|---|---|---|---|---|---|---|
| ATG | AGG | TGT | CTC | GTG | GTG | CTA | CTT | GCT | GTC | TTC | GCT | CTC |
| 24 | | | | | | | | | | | | |
| TCC | CAA | GGC | GCT | GAG | ATC | ACC | AGG | ATC | CCT | CTG | TAC | AAA |
| 63 | | | 72 | | | | | | | | | |
| GGC | AAG | TCT | CTG | AGG | AAG | GCG | CTG | AAG | GAG | CAT | GGG | CTT |
| 102 | | | | | | | 123 | | | | | |
| CTG | GAG | GAC | TTC | CTG | CAG | AAA | CAG | CAG | TAT | GGC | ATC | AGC |
| 141 | | | | 153 | | | | | | | | |
| AGC | AAG | TAC | TCC | GGC | TTC | GGG | GAG | GTG | GCC | AGC | GTG | CCC |
| 180 | 183 | | | | | 198 | | | | | | |
| CTG | ACC | AAC | TAC | CTG | GAT | AGT | CAG | TAC | TTT | GGG | AAG | ATC |
| 219 | | | | | | | | 243 | | | | |
| TAC | CTC | GGG | ACC | CCG | CCC | CAG | GAG | TTC | ACC | GTG | CTG | TTT |
| 258 | | | | | | | | | | | | |
| GAC | ACT | GGC | TCC | TCT | GAC | TTC | TGG | GTA | CCC | TCT | ATC | TAC |
| 297 | | 303 | | | | | | | | | | |
| TGC | AAG | AGC | AAT | GCC | TGC | AAA | AAC | CAC | CAG | CGC | TTC | GAC |
| 336 | | | | | | | | | 363 | | | |
| CCG | AGA | AAG | TCG | TCC | ACC | TTC | CAG | AAC | CTG | GGC | AAG | CCC |
| 375 | | | | | | | | | | | | |
| CTG | TCT | ATC | CAC | TAC | GGG | ACA | GGC | AGC | ATG | CAG | GGC | ATC |
| 414 | | | 423 | | | | | | | | | |
| CTG | GGC | TAT | GAC | ACC | GTC | ACT | GTC | TCC | AAC | ATT | GTG | GAC |
| 453 | | | | | | | | | | 483 | | |
| ATC | CAG | CAG | ACA | GTA | GGC | CTG | AGC | ACC | CAG | GAG | CCC | GGG |
| 492 | | | | | | | | | | | | |
| GAC | GTC | TTC | ACC | TAT | GCC | GAA | TTC | GAC | GGG | ATC | CTG | GGG |
| 531 | | | | 543 | | | | | | | | |
| ATG | GCC | TAC | CCC | TCG | CTC | GCC | TCA | GAG | TAC | TCG | ATA | CCC |
| 570 | | | | | | | | | | | 603 | |
| GTG | TTT | GAC | AAC | ATG | ATG | AAC | AGG | CAC | CTG | GTG | GCC | CAA |
| 609 | | | | | | | | | | | | |

GAC CTG TTC TCG GTT TAC ATG GAC AGG AAT GGC CAG GAG
648 (GAC) 663 (TAC) 675 (AAT)

AGC ATG CTC ACG CTG GGG GCC ATC GAC CCG TCC TAC TAC
687 (AGC) 723 (TAC)

ACA GGG TCC CTG CAT TGG GTG CCC GTG ACA GTG CAG CAG
726 (ACA)

TAC TGG CAG TTC ACT GTG GAC AGT GTC ACC ATC AGC GGT
765 (TAC) 783 (GAC)

GTG GTT GTG GCC TGT GAG GGT GGC TCT CAG GCC ATC CTG
804 (GTG)

GAC ACG GGC ACC TCC AAG CTG GTC GGG CCC AGC AGC GAC
843 (GAC)

ATC CTC AAC ATC CAG CAG GCC ATT GGA GCC ACA CAG AAC
882 (ATC) 903 (ATT)

CAG TAC GGT GAG TTT GAC ATC GAC TGC GAC AAC CTG AGC
921 (CAG) 928 (GGT)

TAC ATG CCC ACT GTG GTC TTT GAG ATC AAT GGC AAA ATG
960 (TAC)

TAC CCA CTG ACC CCC TCC GCC TAT ACC AGC CAG GAC CAG
999 (TAC) 1023 (ACC)

GGC TTC TGT ACC AGT GCC TTC CAG AGT GAA AAT CAT TCC
1038 (GGC)

CAG AAA TGG ATC CTG GGG GAT GTT TTC ATC CGA GAG TAT
1077 (CAG) 1083 (TGG)

TAC AGC GTC TTT GAC AGG GCC AAC AAC CTC GTG GGG CTG
1116 (TAC) 1143 (CTC)

GCC AAA GCC ATC,
1155 (GCC) 1166 (ATC)

(b) the polynucleotide 24—1166, which is the same as that of (a), except that the nucleotide A at position 675 is replaced by G,

(c) the polynucleotide 24—1166, which is the same as that of (a), except that the nucleotide G at position 928 is replaced by A, and

(d) the polynucleotide 24—1166, which is the same as that of (a), except that the nucleotide A at position 675 is replaced by G and the nucleotide G at position 928 is replaced by A.

3. A recombinant plasmid according to Claim 1, wherein the plus strand of the prochymosin ds-rDNA of (1) is selected from the following sequences

(a) the polynucleotide 72—1166 given in Claim 2(a),

(b) the polynucleotide 72—1166 given in Claim 2(b),
(c) the polynucleotide 72—1166 given in Claim 2(c) and
(d) the polynucleotide 72—1166 given in Claim 2(d).

4. A recombinant plasmid according to Claim 1, wherein the plus strand of the pseudochymosin ds-rDNA of (1) is selected from the following sequences
(a) the polynucleotide 153—1166 given in Claim 2(a),
(b) the polynucleotide 153—1166 given in Claim 2(b),
(c) the polynucleotide 153—1166 given in Claim 2(c) and
(d) the polynucleotide 153—1166 given in Claim 2(d).

5. A recombinant plasmid according to Claim 1, wherein the plus stand of the chymosin ds-rDNA of (1) is selected from the following sequences
(a) the polynucleotide 198—1166 given in Claim 2(a),
(b) the polynucleotide 198—1166 given in Claim 2(b),
(c) the polynucleotide 193—1166 given in Claim 2(c), and
(d) the polynucleotide 198—1166 given in Claim 2(d).

6. A recombinant plasmid according to Claim 1, comprising a regulon consisting of a double lac UV5 system.

7. A recombinant plasmid according to Claim 1, comprising a regulon consisting of at least one modified tryptophan system, wherein the information coding for the trp attenuator protein is eliminated.

8. A recombinant plasmid according to Claim 7, comprising two modified trp regulons linked in a head to tail fashion.

9. A recombinant plasmid according to Claim 1, comprising a regulon consisting of at least one modified promotor/ribosome-binding site of gene VIII of bacteriophage M13.

10. A recombinant plasmid according to Claim 1, comprising a thermosensitive replication mutant derived from the cloacin DF 13 plasmid pVU 208.

11. A recombinant plasmid according to Claim 1, selected from pUR 1521 (ATCC 39120), pUR 1533 (ATCC 39121), pUR 1734 (ATCC 39198) and pUR 1832 (ATCC 39197).

12. A bacterial culture comprising *E. coli* cells transformed with plasmids according to any one of Claims 1—11.

13. A process for producing preprochymosin or any of its maturation forms prochymosin, pseudochymosin or chymosin, which comprises culturing a bacterial culture according to Claim 12 under a selection pressure, and collecting the preprochymosin or a maturation form thereof.

14. Micro-organisms, which are transformed by incorporation of
(a) a ds-rDNA coding for preprochymosin, prochymosin, pseudochymosin or chymosin,
(b) a translational stop codon bound to the 3'-end of the plus strand of the ds-rDNA of (a),
(c) a selective marker and preferably a replication site adapted to said micro-organisms,
(d) an expression regulon suitable for said micro-organisms upstream of the plus strand of the ds-rDNA of (a), and
(e) when the ds-rDNA codes for prochymosin, pseudochymosin or chymosin, a translational initiation ATG-triplet bound to the 5'-end of the plus strand of the ds-rDNA of (a), whereby the elements a, b, d and optionally e are present in the order d-(e)-a-b.

15. Micro-organisms according to Claim 14, wherein the ds-rDNA is selected from the ds-rDNA sequences specified in Claims 2(a)—(d), 3(a)—(d), 4(a)—(d) and 5(a)—(d).

16. Micro-organisms according to Claim 14 or 15, wherein the ds-rDNA codes for preprochymosin.

17. Micro-organisms according to any one of Claims 14—16, wherein the micro-organism is non-toxic and edible and is selected from the group consisting of streptococci or lactobacilli and micro-organisms of *Bacillus* or yeast origin.

18. A process for producing preprochymosin or any of its maturation forms prochymosin, pseudochymosin or chymosin, which comprises culturing a micro-organism according to any one of Claims 14—17, optionally under a selection pressure, and collecting the preprochymosin or a maturation form thereof.

**Claims for the Contracting State: AT**

1. A process for preparing a recombinant plasmid which comprises combining, by recombinant DNA techniques, the following elements in the order given
(1) a ds-rDNA coding for preprochymosin, prochymosin, pseudochymosin or chymosin,
(2) a translational stop codon bound to the 3'-end of the plus strand of the ds-rDNA of (1),
(3) both an *E. coli* replication site and a selective marker,
(4) an *E. coli* expression regulon upstream of the plus strand of the ds-rDNA of (1), and
(5) when the ds-rDNA codes for prochymosin, pseudochymosin or chymosin, a translational initiation ATG-triplet bound to the 5'-end of the plus strand of the ds-rDNA of (1).

2. A process according to Claim 1, wherein the plus strand of the preprochymosin ds-rDNA of (1) is selected from the following sequences
(a) the polynucleotide 24—1166

| | | | | | | | | | | | | |
|---|---|---|---|---|---|---|---|---|---|---|---|---|
| ATG | AGG | TGT | CTC | GTG | GTG | CTA | CTT | GCT | GTC | TTC | GCT | CTC |
| 24 | | | | | | | | | | | | |
| TCC | CAA | GGC | GCT | GAG | ATC | ACC | AGG | ATC | CCT | CTG | TAC | AAA |
| 63 | | | 72 | | | | | | | | | |
| GGC | AAG | TCT | CTG | AGG | AAG | GCG | CTG | AAG | GAG | CAT | GGG | CTT |
| 102 | | | | | | | 123 | | | | | |
| CTG | GAG | GAC | TTC | CTG | CAG | AAA | CAG | CAG | TAT | GGC | ATC | AGC |
| 141 | | | | 153 | | | | | | | | |
| AGC | AAG | TAC | TCC | GGC | TTC | GGG | GAG | GTG | GCC | AGC | GTG | CCC |
| 180 | 183 | | | | | 198 | | | | | | |
| CTG | ACC | AAC | TAC | CTG | GAT | AGT | CAG | TAC | TTT | GGG | AAG | ATC |
| 219 | | | | | | | | 243 | | | | |
| TAC | CTC | GGG | ACC | CCG | CCC | CAG | GAG | TTC | ACC | GTG | CTG | TTT |
| 258 | | | | | | | | | | | | |
| GAC | ACT | GGC | TCC | TCT | GAC | TTC | TGG | GTA | CCC | TCT | ATC | TAC |
| 297 | | 303 | | | | | | | | | | |
| TGC | AAG | AGC | AAT | GCC | TGC | AAA | AAC | CAC | CAG | CGC | TTC | GAC |
| 336 | | | | | | | | | 363 | | | |
| CCG | AGA | AAG | TCG | TCC | ACC | TTC | CAG | AAC | CTG | GGC | AAG | CCC |
| 375 | | | | | | | | | | | | |
| CTG | TCT | ATC | CAC | TAC | GGG | ACA | GGC | AGC | ATG | CAG | GGC | ATC |
| 414 | | | 423 | | | | | | | | | |
| CTG | GGC | TAT | GAC | ACC | GTC | ACT | GTC | TCC | AAC | ATT | GTG | GAC |
| 453 | | | | | | | | | | 483 | | |
| ATC | CAG | CAG | ACA | GTA | GGC | CTG | AGC | ACC | CAG | GAG | CCC | GGG |
| 492 | | | | | | | | | | | | |
| GAC | GTC | TTC | ACC | TAT | GCC | GAA | TTC | GAC | GGG | ATC | CTG | GGG |
| 531 | | | | 543 | | | | | | | | |
| ATG | GCC | TAC | CCC | TCG | CTC | GCC | TCA | GAG | TAC | TCG | ATA | CCC |
| 570 | | | | | | | | | | | 603 | |
| GTG | TTT | GAC | AAC | ATG | ATG | AAC | AGG | CAC | CTG | GTG | GCC | CAA |
| 609 | | | | | | | | | | | | |

GAC CTG TTC TCG GTT TAC ATG GAC AGG AAT GGC CAG GAG
648 (GAC) 663 (TAC) 675 (AAT)

AGC ATG CTC ACG CTG GGG GCC ATC GAC CCG TCC TAC TAC
687 (AGC) 723 (TAC)

ACA GGG TCC CTG CAT TGG GTG CCC GTG ACA GTG CAG CAG
726 (ACA)

TAC TGG CAG TTC ACT GTG GAC AGT GTC ACC ATC AGC GGT
765 (TAC) 783 (GAC)

GTG GTT GTG GCC TGT GAG GGT GGC TCT CAG GCC ATC CTG
804 (GTG)

GAC ACG GGC ACC TCC AAG CTG GTC GGG CCC AGC AGC GAC
843 (GAC)

ATC CTC AAC ATC CAG CAG GCC ATT GGA GCC ACA CAG AAC
882 (ATC) 903 (ATT)

CAG TAC GGT GAG TTT GAC ATC GAC TGC GAC AAC CTG AGC
921 (CAG) 928 (GGT)

TAC ATG CCC ACT GTG GTC TTT GAG ATC AAT GGC AAA ATG
960 (TAC)

TAC CCA CTG ACC CCC TCC GCC TAT ACC AGC CAG GAC CAG
999 (TAC) 1023 (ACC)

GGC TTC TGT ACC AGT GCC TTC CAG AGT GAA AAT CAT TCC
1038 (GGC)

CAG AAA TGG ATC CTG GGG GAT GTT TTC ATC CGA GAG TAT
1077 (CAG) 1083 (TGG)

TAC AGC GTC TTT GAC AGG GCC AAC AAC CTC GTG GGG CTG
1116 (TAC) 1143 (CTC)

GCC AAA GCC ATC,
1155 (GCC) 1166 (ATC)

(b) the polynucleotide 24—1166, which is the same as that of (a), except that the nucleotide A at position 675 is replaced by G,

(c) the polynucleotide 24-1166, which is the same as that of (a), except that the nucleotide G at position 928 is replaced by A, and

(d) the polynucleotide 24-1166, which is the same as that of (a), except that the nucleotide A at position 675 is replaced by G and the nucleotide G at position 928 is replaced by A.

3. A process according to Claim 1, wherein the plus strand of the prochymosin ds-rDNA of (1) is selected from the following sequences

(a) the polynucleotide 72—1166 given in Claim 2(a),

(b) the polynucleotide 72—1166 given in Claim 2(b),
(c) the polynucleotide 72—1166 given in Claim 2(c) and
(d) the polynucleotide 72—1166 given in Claim 2(d).

4. A process according to Claim 1, wherein the plus strand of the pseudochymosin ds-rDNA of (1) is selected from the following sequences
(a) the polynucleotide 153—1166 given in Claim 2(a),
(b) the polynucleotide 153—1166 given in Claim 2(b),
(c) the polynucleotide 153—1166 given in Claim 2(c), and
(d) the polynucleotide 153—1166 given in Claim 2(d).

5. A process according to Claim 1, wherein the plus strand of the chymosin ds-rDNA of (1) is selected from the following sequences
(a) the polynucleotide 198—1166 given in Claim 2(a),
(b) the polynucleotide 198—1166 given in Claim 2(b),
(c) the polynucleotide 198—1166 given in Claim 2(c) and
(d) the polynucleotide 198—1166 given in Claim 2(d).

6. A process according to Claim 1, wherein the regulon of (4) is a double lac UV5 system.

7. A process according to Claim 1, wherein the regulon of (4) is at least one modified tryptophan system, wherein the information coding for the trp attenuator protein is eliminated.

8. A process according to Claim 7, wherein two modified trp regulons are linked in a head to tail fashion.

9. A process according to Claim 1, wherein the regulon of (4) is at least one modified promotor/ribosome-binding site of gene VIII of bacteriophage M13.

10. A process according to Claim 1, wherein the plasmid comprises a thermosensitive replication mutant derived from the cloacin DF 13 plasmid pVU 208.

11. A process according to Claim 1, wherein the recombinant plasmid is selected from pUR 1521 (ATCC 39120), pUR 1533 (ATCC 39121), pUR 1734 (ATCC 39198) and pUR 1832 (ATCC 39197).

12. A process for preparing a bacterial culture which comprises transforming *E. coli* cells with plasmids prepared by a process as claimed in any one of Claims 1—11.

13. A process for producing preprochymosin or any of its maturation forms prochymosin, pseudochymosin or chymosin, which comprises culturing a bacterial culture prepared by a process as claimed in Claim 12 under a selection pressure, and collecting the preprochymosin or a maturation form thereof.

14. A process for preparing transformed micro-organisms, which comprises incorporation in the micro-organisms
(a) ds-rDNA coding for preprochymosin, prochymosin, pseudochymosin or chymosin,
(b) a translational stop codon bound to the 3'-end of the plus strand of the ds-rDNA of (a),
(c) a selective marker and preferably a replication site adapted to said micro-organisms,
(d) an expression regulon suitable for said micro-organisms upstream of the plus strand of the ds-rDNA of (a), and
(e) when the ds-rDNA codes for prochymosin, pseudochymosin or chymosin, a translational initiation ATG-triplet bound to the 5'-end of the plus strand of the ds-rDNA of (a), whereby the elements a, b, d and optionally e are arranged in the order d-(e)-a-b.

15. A process according to Claim 14, wherein the ds-rDNA is selected from the ds-rDNA sequences specified in Claims 2(a)—(d), 3(a)—(d), 4(a)—(d) and 5(a)—(d).

16. A process according to Claim 14 or 15, wherein the ds-rDNA codes for preprochymosin.

17. A process according to any one of Claims 14—16, wherein the micro-organism is non-toxic and edible and is selected from the group consisting of streptococci or lactobacilli and micro-organisms of *Bacillus* or yeast origin.

18. A process for producing preprochymosin or any of its maturation forms prochymosin, pseudochymosin or chymosin, which comprises culturing a micro-organism prepared by a process as claimed in any one of Claims 14—17, optionally under a selection pressure, and collecting the preprochymosin or a maturation form thereof.

**Patentansprüche für die Vertragsstaaten: BE, CH, DE, FR, GB, IT, LI, NL, SE**

1. Rekombinantes Plasmid, enthaltend die folgenden Elemente in der angegebenen Reihenfolge
(1) eine ds-rDNA, Preprochymosin, Prochymosin, Pseudochymosin oder Chymosin kodierend,
(2) ein translationales Unterbrechungskodon, gebunden an das 3'-Ende des Plus-Stranges der ds-rDNA von (1),
(3) sowohl eine E. coli-Replikationsstelle als auch einen selektiven Marker,
(4) ein E. coli-Expressionsregulon stromaufwärts vom Plus-Strang der ds-rDNA von (1) und,
(5) wenn die ds-rDNA Prochymosin, Pseudochymosin oder Chymosin kodiert, eine translationales Initiations-ATG-Triplett, gebunden an das 5'-Ende des Plus-Stranges der ds-rDNA von (1).

2. Rekombinantes Plasmid gemäß Anspruch 1, worin der Plus-Strang der Preprochymosin-ds-rDNA von (1) augewählt ist aus den folgenden Sequenzen
(a) dem Polynukleotid 24—1166

ATG AGG TGT CTC GTG GTG CTA CTT GCT GTC TTC GCT CTC
|
24

TCC CAA GGC GCT GAG ATC ACC AGG ATC CCT CTG TAC AAA
| |
63 72

GGC AAG TCT CTG AGG AAG GCG CTG AAG GAG CAT GGG CTT
| |
102 123

CTG GAG GAC TTC CTG CAG AAA CAG CAG TAT GGC ATC AGC
| |
141 153

AGC AAG TAC TCC GGC TTC GGG GAG GTG GCC AGC GTG CCC
| | |
180 183 198

CTG ACC AAC TAC CTG GAT AGT CAG TAC TTT GGG AAG ATC
| |
219 243

TAC CTC GGG ACC CCG CCC CAG GAG TTC ACC GTG CTG TTT
|
258

GAC ACT GGC TCC TCT GAC TTC TGG GTA CCC TCT ATC TAC
| |
297 303

TGC AAG AGC AAT GCC TGC AAA AAC CAC CAG CGC TTC GAC
| |
336 363

CCG AGA AAG TCG TCC ACC TTC CAG AAC CTG GGC AAG CCC
|
375

CTG TCT ATC CAC TAC GGG ACA GGC AGC ATG CAG GGC ATC
| |
414 423

CTG GGC TAT GAC ACC GTC ACT GTC TCC AAC ATT GTG GAC
| |
453 483

ATC CAG CAG ACA GTA GGC CTG AGC ACC CAG GAG CCC GGG
|
492

GAC GTC TTC ACC TAT GCC GAA TTC GAC GGG ATC CTG GGG
| |
531 543

ATG GCC TAC CCC TCG CTC GCC TCA GAG TAC TCG ATA CCC
| |
570 603

GTG TTT GAC AAC ATG ATG AAC AGG CAC CTG GTG GCC CAA
|
609

| | | | | | | | | | | | | |
|---|---|---|---|---|---|---|---|---|---|---|---|---|
| GAC | CTG | TTC | TCG | GTT | TAC | ATG | GAC | AGG | AAT | GGC | CAG | GAG |
| 648 | | | | | 663 | | | | 675 | | | |
| AGC | ATG | CTC | ACG | CTG | GGG | GCC | ATC | GAC | CCG | TCC | TAC | TAC |
| 687 | | | | | | | | | | | | 723 |
| ACA | GGG | TCC | CTG | CAT | TGG | GTG | CCC | GTG | ACA | GTG | CAG | CAG |
| 726 | | | | | | | | | | | | |
| TAC | TGG | CAG | TTC | ACT | GTG | GAC | AGT | GTC | ACC | ATC | AGC | GGT |
| 765 | | | | | | 783 | | | | | | |
| GTG | GTT | GTG | GCC | TGT | GAG | GGT | GGC | TCT | CAG | GCC | ATC | CTG |
| 804 | | | | | | | | | | | | |
| GAC | ACG | GGC | ACC | TCC | AAG | CTG | GTC | GGG | CCC | AGC | AGC | GAC |
| 843 | | | | | | | | | | | | |
| ATC | CTC | AAC | ATC | CAG | CAG | GCC | ATT | GGA | GCC | ACA | CAG | AAC |
| 882 | | | | | | | 903 | | | | | |
| CAG | TAC | GGT | GAG | TTT | GAC | ATC | GAC | TGC | GAC | AAC | CTG | AGC |
| 921 | | 928 | | | | | | | | | | |
| TAC | ATG | CCC | ACT | GTG | GTC | TTT | GAG | ATC | AAT | GGC | AAA | ATG |
| 960 | | | | | | | | | | | | |
| TAC | CCA | CTG | ACC | CCC | TCC | GCC | TAT | ACC | AGC | CAG | GAC | CAG |
| 999 | | | | | | | | 1023 | | | | |
| GGC | TTC | TGT | ACC | AGT | GCC | TTC | CAG | AGT | GAA | AAT | CAT | TCC |
| 1038 | | | | | | | | | | | | |
| CAG | AAA | TGG | ATC | CTG | GGG | GAT | GTT | TTC | ATC | CGA | GAG | TAT |
| 1077 | | 1083 | | | | | | | | | | |
| TAC | AGC | GTC | TTT | GAC | AGG | GCC | AAC | AAC | CTC | GTG | GGG | CTG |
| 1116 | | | | | | | | | 1143 | | | |
| GCC | AAA | GCC | ATC | | | | | | | | | |
| 1155 | | | 1166 | | | | | | | | | |

(b) dem Polynukleotid 24—1166, das das gleiche wie das von (a) ist mit der Ausnahme, daß das Nukleotid A in Position 675 durch G ersetzt ist,

(c) dem Polynukleotid 24—1166, das das gleiche wie das von (a) ist mit der Ausnahme, daß das Nukleotid G in Position 928 durch A ersetzt ist, und

(d) dem Polynukleotid 24—1166, das das gleiche wie das von (a) ist mit der Ausnahme, daß das Nukleotid A in Position 675 durch G ersetzt ist und das Nukleotid G in Position 928 durch A ersetzt ist.

3. Rekombinantes Plasmid nach Anspruch 1, worin der Plus-Strang der Prochymosin ds-rDNA von (1) ausgewählt ist aus den folgenden Sequenzen

(a) dem Polynukleotid 72—1166, angegeben in Anspruch 2(a),

(b) dem Polynukleotid 72—1166, angegeben in Anspruch 2(b),
(c) dem Polynukleotid 72—1166, angegeben in Anspruch 2(c) und
(d) dem Polynukleotid 72—1166, angegeben in Anspruch 2(d).

4. Rekombinantes Plasmid nach Anspruch 1, worin der Plus-Strang der Pseudochymosin ds-rDNA von (1) ausgewählt ist aus dem folgenden Sequenzen
(a) dem Polynukleotid 153—1166, angegeben in Anspruch 2(a),
(b) dem Polynukleotid 153—1166, angegeben in Anspruch 2(b),
(c) dem Polynukleotid 153—1166, angegeben in Anspruch 2(c) und
(d) dem Polynukleotid 153—1166, angegeben in Anspruch 2(d).

5. Rekombinantes Plasmid nach Anspruch 1, worin der Plus-Strang der Chymosin-ds-rDNA von (1) ausgewählt ist aus den folgenden Sequenzen
(a) dem Polynukleotid 193—1166, angegeben in Anspruch 2(a),
(b) dem Polynukleotid 198—1166, angegeben in Anspruch 2(b),
(c) dem Polynukleotid 198—1166, angegeben in Anspruch 2(c) und
(d) dem Polynukleotid 193—1166, angegeben in Anspruch 2(d).

6. Rekombinantes Plasmid nach Anspruch 1, enthaltend ein Regulon, das aus einem Doppel-lac-UV5-System besteht.

7. Rekombinantes Plasmid nach Anspruch 1; enthaltend ein Regulon, das aus mindestens einem modifizierten Tryptophansystem besteht, worin die das trp-Attenuator-Protein kodierende Information eliminiert ist.

8. Rekombinantes Plasmid nach Anspruch 7, enthaltend zwei modifizierte trp-Regulons, die in Kopf-an-Schwanz-Weise gebunden sind.

9. Rekombinantes Plasmid nach Anspruch 1, enthaltend ein Regulon, das aus mindestens einer modifizierten Promotor/Ribosomen bindenden Stelle von Gen VIII des Bateriophagen M13 besteht.

10. Rekombinantes Plasmid nach Anspruch 1, enthaltend eine wärmeempfindliche Replikationsmutante, abgeleitet vom Cloacin-DF-13-Plasmid pVU 208.

11. Rekombinantes Plasmid nach Anspruch 1, ausgewählt aus pUR 1521 (ATCC 39120), pUR 1533 (ATCC 39121), pUR 1734 (ATCC 39198) und pUR 1832 (ATCC 39197).

12. Eine Bakterienkultur, umfassend E. coli-Zellen, die mit Plasmiden gemäß irgendeinem der Ansprüche 1 bis 11 transformiert sind.

13. Verfahren zur Herstellung von Preprochymosin oder irgendeiner von dessen Reifungsformen Prochymosin, Pseudochymosin oder Chymosin, das die Züchtung einer Bakterienkultur gemäß Anspruch 12 unter einem Selektionsdruck und Sammeln von Preprochymosin oder einer Reifungsform desselben umfaßt.

14. Mikroorganismen, die transformiert sind durch Einverleibung von
(a) einer ds-rDNA, die Preprochymosin, Prochymosin, Pseudochymosin oder Chymosin kodiert,
(b) einem translationalen Unterbrechungskodon, gebunden an das 3'-Ende des Plus-Stranges der ds-rDNA von (a),
(c) einem selektiven Marker und vorzugsweise einer an diese Mikroorganismen angepaßten Replikationsstelle,
(d) einem für diese Mikroorganismen geeigneten Expressionsregulon stromaufwärts vom Plus-Strang der ds-rDNA von (a) und,
(e) wenn die ds-rDNA Prochymosin, Pseudochymosin oder Chymosin kodiert, einem translationalen Initiations-ATG-Triplett, gebunden an das 5'-Ende des Plusstranges der ds-rDNA von (a),
wodurch die Elemente a, b, d und wahlweise e in der Reihenfolge d-(e)-a-b anwesend sind.

15. Mikroorganismen nach Anspruch 14, worin die ds-rDNA ausgewählt ist aus den ds-rDNA-Sequenzen, die in Anspruch 2(a)—(d), 3(a)—(d), 4(a)—(d) und 5(a)—(d) angegeben sind.

16. Mikroorganismen nach Anspruch 14 oder 15, worin die ds-rDNA Preprochymosin kodiert.

17. Mikroorganismen nach einem der Ansprüche 14 bis 16, worin der Mikroorganismus nicht-toxisch und eßbar ist und ausgewählt ist aus der aus Streptococci oder Lactobacilli und Mikroorganismen von *Bacillus* oder Hefeursprung bestehenden Gruppe.

18. Verfahren zur Herstellung von Preprochymosin oder irgendeiner von dessen Reifungsformen Prochymosin, Pseudochymosin oder Chymosin, das die Züchtung eines Mikroorganismus gemäß irgendeinem der Ansprüche 14 bis 17, gegebenenfalls unter einem Selektionsdruck, und Sammeln von Preprochymosin oder einer Reifungsform desselben umfaßt.

**Patentansprüche für den Vertragsstaat: AT**

1. Verfahren zur Herstellung eines rekombinanten Plasmids, das umfaßt das Kombinieren der folgenden Elemente in der angegebenen Reihenfolge durch rekombinante DNA-Techniken:
(1) einer ds-rDNA, die Preprochymosin, Prochymosin, Pseudochymosin oder Chymosin kodiert,
(2) eines translationalen Unterbrechungskodons, gebunden an das 3'-Ende des Plus-Stranges der ds-rDNA von (1),
(3) sowohl einer E. coli-Replikationsstelle als auch eines selektiven Markers,
(4) eines E. coli-Expressionsregulons stromaufwärts vom Plus-Strang der ds-rDNA von (1) und,

(5) wenn die ds-rDNA Prochymosin, Pseudochymosin oder Chymosin kodiert, eines translationalen Initiations-ATG-Tripletts, gebunden an das 5'-Ende des Plus-Stranges der ds-rDNA von (1).

2. Verfahren nach Anspruch 1, worin der Plusstrang der Preprochymosin-ds-rDNA von (1) ausgewählt ist aus den folgenden Sequenzen

(a) dem Polynukleotid 24—1166

| | | | | | | | | | | | | |
|---|---|---|---|---|---|---|---|---|---|---|---|---|
| ATG | AGG | TGT | CTC | GTG | GTG | CTA | CTT | GCT | GTC | TTC | GCT | CTC |
| 24 | | | | | | | | | | | | |
| TCC | CAA | GGC | GCT | GAG | ATC | ACC | AGG | ATC | CCT | CTG | TAC | AAA |
| 63 | | | 72 | | | | | | | | | |
| GGC | AAG | TCT | CTG | AGG | AAG | GCG | CTG | AAG | GAG | CAT | GGG | CTT |
| 102 | | | | | | | 123 | | | | | |
| CTG | GAG | GAC | TTC | CTG | CAG | AAA | CAG | CAG | TAT | GGC | ATC | AGC |
| 141 | | | | 153 | | | | | | | | |
| AGC | AAG | TAC | TCC | GGC | TTC | GGG | GAG | GTG | GCC | AGC | GTG | CCC |
| 180 | 183 | | | | | 198 | | | | | | |
| CTG | ACC | AAC | TAC | CTG | GAT | AGT | CAG | TAC | TTT | GGG | AAG | ATC |
| 219 | | | | | | | | 243 | | | | |
| TAC | CTC | GGG | ACC | CCG | CCC | CAG | GAG | TTC | ACC | GTG | CTG | TTT |
| 258 | | | | | | | | | | | | |
| GAC | ACT | GGC | TCC | TCT | GAC | TTC | TGG | GTA | CCC | TCT | ATC | TAC |
| 297 | | 303 | | | | | | | | | | |
| TGC | AAG | AGC | AAT | GCC | TGC | AAA | AAC | CAC | CAG | CGC | TTC | GAC |
| 336 | | | | | | | | | 363 | | | |
| CCG | AGA | AAG | TCG | TCC | ACC | TTC | CAG | AAC | CTG | GGC | AAG | CCC |
| 375 | | | | | | | | | | | | |
| CTG | TCT | ATC | CAC | TAC | GGG | ACA | GGC | AGC | ATG | CAG | GGC | ATC |
| 414 | | | 423 | | | | | | | | | |
| CTG | GGC | TAT | GAC | ACC | GTC | ACT | GTC | TCC | AAC | ATT | GTG | GAC |
| 453 | | | | | | | | | | 483 | | |
| ATC | CAG | CAG | ACA | GTA | GGC | CTG | AGC | ACC | CAG | GAG | CCC | GGG |
| 492 | | | | | | | | | | | | |
| GAC | GTC | TTC | ACC | TAT | GCC | GAA | TTC | GAC | GGG | ATC | CTG | GGG |
| 531 | | | | 543 | | | | | | | | |
| ATG | GCC | TAC | CCC | TCG | CTC | GCC | TCA | GAG | TAC | TCG | ATA | CCC |
| 570 | | | | | | | | | | | 603 | |

GTG TTT GAC AAC ATG ATG AAC AGG CAC CTG GTG GCC CAA
609

GAC CTG TTC TCG GTT TAC ATG GAC AGG AAT GGC CAG GAG
648 663 675

AGC ATG CTC ACG CTG GGG GCC ATC GAC CCG TCC TAC TAC
687 723

ACA GGG TCC CTG CAT TGG GTG CCC GTG ACA GTG CAG CAG
726

TAC TGG CAG TTC ACT GTG GAC AGT GTC ACC ATC AGC GGT
765 783

GTG GTT GTG GCC TGT GAG GGT GGC TCT CAG GCC ATC CTG
804

GAC ACG GGC ACC TCC AAG CTG GTC GGG CCC AGC AGC GAC
843

ATC CTC AAC ATC CAG CAG GCC ATT GGA GCC ACA CAG AAC
882 903

CAG TAC GGT GAG TTT GAC ATC GAC TGC GAC AAC CTG AGC
921 928

TAC ATG CCC ACT GTG GTC TTT GAG ATC AAT GGC AAA ATG
960

TAC CCA CTG ACC CCC TCC GCC TAT ACC AGC CAG GAC CAG
999 1023

GGC TTC TGT ACC AGT GCC TTC CAG AGT GAA AAT CAT TCC
1038

CAG AAA TGG ATC CTG GGG GAT GTT TTC ATC CGA GAG TAT
1077 1083

TAC AGC GTC TTT GAC AGG GCC AAC AAC CTC GTG GGG CTG
1116 1143

GCC AAA GCC ATC
1155 1166

(b) dem Polynukleotid 24—1166, das das gleiche wie das von (a) ist mit der Ausnahme, daß das Nukleotid A in Position 675 durch G ersetzt ist,

(c) dem Polynukleotid 24—1166, das das gleiche wie das von (a) ist mit der Ausnahme, daß das Nukleotid G in Position 928 durch A ersetzt ist, und

(d) dem Polynukleotid 24—1166, das das gleiche wie das von (a) ist mit der Ausnahme, daß das Nukleotid A in Position 675 durch G ersetzt ist und das Nukleotid G in Position 928 durch A ersetzt ist.

3. Verfahren nach Anspruch 1, worin der Plus-Strang der Prochymosin-ds-rDNA von (1) ausgewählt ist aus den folgenden Sequenzen

(a) dem Polynukleotid 72—1166, angegeben in Anspruch 2(a),

(b) dem Polynukleotid 72—1166, angegeben in Anspruch 2(b),

(c) dem Polynukleotid 72—1166, angegeben in Anspruch 2(c) und

(d) dem Polynukleotid 72—1166, angegeben in Anspruch 2(d).

4. Verfahren nach Anspruch 1, worin der Plus-Strang der Pseudochymosin-ds-rDNA von (1) ausgewählt ist aus den folgenden Sequenzen

(a) dem Polynukleotid 153—1166, angegeben in Anspruch 2(a),

(b) dem Polynukleotid 153—1166, angegeben in Anspruch 2(b),

(c) dem Polynukleotid 153—1166, angegeben in Anspruch 2(c) und

(d) dem Polynukleotid 153—1166, angegeben in Anspruch 2(d).

5. Verfahren nach Anspruch 1, worin der Plus-Strang der Chymosin-ds-rDNA von (1) ausgewählt ist aus dem folgenden Sequenzen

(a) dem Polynukleotid 198—1166, angegeben in Anspruch 2(a),

(b) dem Polynukleotid 198—1166, angegeben in Anspruch 2(b),

(c) dem Polynukleotid 198—1166, angegeben in Anspruch 2(c) und

(d) dem Polynukleotid 193—1166, angegeben in Anspruch 2(d).

6. Verfahren nach Anspruch 1, worin das Regulon von (4) ein Doppel-lac-UV5-System ist.

7. Verfahren nach Anspruch 1, worin das Regulon von (4) mindestens ein modifiziertes Tryptophansystem ist, worin die das trp-Attenuator-Protein kodierende Information eliminiert ist.

8. Verfahren nach Anspruch 7, worin die beiden modifizierten trp-Regulons in Kopf-an-Schwanz-Weise gebunden sind.

9. Verfahren nach Anspruch 1, worin das Regulon von (4) mindestens eine modifizierte Promotor/Ribosomen bindende Stelle von Gen VIII des Bakteriophagen M13 ist.

10. Verfahren nach Anspruch 1, worin das Plasmid eine wärmeempfindliche Replikationsmutante umfaßt, abgeleitet vom Cloacin-DF-13-Plasmid pVU 208.

11. Verfahren nach Anspruch 1, worin das rekombinante Plasmid ausgewählt ist aus pUR 1521 (ATCC 39120), pUR 1533 (ATCC 39121), pUR 1734 (ATCC 39198) und pUR 1832 (ATCC 39197).

12. Verfahren zur Herstellung einer Bakterienkultur, das die Transformation von E. coli-Zellen mit Plasmiden, die nach einem Verfahren gemäß irgendeinem der Ansprüche 1 bis 11 hergestellt sind, umfaßt.

13. Verfahren zur Herstellung von Preprochymosin oder irgendeiner von dessen Reifungsformen Prochymosin, Pseudochymosin oder Chymosin, das die Züchtung einer Bakterienkultur, hergestellt nach einem Verfahren gemäß Anspruch 12 unter einem Selektionsdruck, und Sammeln des Preprochymosins oder einer Reifungsform desselben umfaßt.

14. Verfahren zur Herstellung transformierter Mikroorganismen, das umfaßt die Einverleibung in den Mikroorganismus

(a) von ds-rDNA, die Preprochymosin, Prochymosin, Pseudochymosin oder Chymosin kodiert,

(b) eines translationalen Unterbrechungskodons, gebunden an das 3'-Ende des Plus-Stranges der ds-rDNA von (a),

(c) eines selektiven Markers und vorzugsweise einer an diese Mikroorganismen angepaßten Replikationsstelle,

(d) eines für diese Mikroorganismen geeigneten Expressionsregulons stromaufwärts vom Plus-Strang der ds-rDNA von (a), und,

(e) wenn die ds-rDNA Prochymosin, Pseudochymosin oder Chymosin kodiert, eines translationalen Initiations-ATG-Tripletts, gebunden an das 5'-Ende des Plus-Stranges der ds-rDNA von (a),

wobei die Elemente a, b, d und gegebenenfalls e in der Reihenfolge d-(e)-a-b angeordnet sind.

15. Verfahren nach Anspruch 14, worin die ds-rDNA ausgewählt ist aus den in Ansprüchen 2(a)—(d), 3(a)—(d), 4(a)—(d) und 5(a)—(d) angegebenen ds-rDNA-Sequenzen sind.

16. Verfahren nach Anspruch 14 oder 15, worin die ds-rDNA Preprochymosin kodiert.

17. Verfahren nach irgendeinem der Ansprüche 14 bis 16, worin der Mikroorganismus nicht-toxisch und eßbar ist und ausgewählt ist aus der aus Streptococci oder Lactobacilli und Mikroorganismen von Bacillus- oder Hefeursprung bestehenden Gruppe.

18. Verfahren zur Herstellung von Preprochymosin oder irgendeiner von dessen Reifungsformen Prochymosin, Pseudochymosin oder Chymosin, das die Züchtung eines Mikroorganismus, hergestellt nach einem Verfahren gemäß irgendeinem der Ansprüche 14 bis 17, gegebenenfalls unter einem Selektionsdruck, und Sammeln von Preprochymosin oder einer Reifungsform desselben umfaßt.

**Revendications pour les Etats Contractants: BE, CH, DE, FR, GB, LI, NL, SE**

1. Plasmide recombinant comprenant les éléments suivants dans l'ordre indiqué

(1) ADNr-ds codant pour la préprochymosine, la prochymosine, la pseudochymosine ou la chymosine,

(2) codon de terminaison de traduction lié à l'extrémité 3' du brin plus de l'ADNr-ds de (1),

(3) à la fois un site de réplication d'*E. coli* et un marqueur sélectif,

(4) régulon d'expression d'*E. coli* en amont du brin plus de l'ADNr-ds de (1), et

(5) lorsque l'ADNr-ds code pour la prochymosine, la pseudochymosine ou la chymosine, un triplet ATG d'initiation de traduction lié à l'extrémité 5' du brin plus de l'ADNr-ds de (1).

2. Plasmide recombinant selon la Revendication 1, dans lequel le brin plus de l'ADNr-ds de la préprochymosine de (1) est choisi à partir des séquences suivantes

(a) le polynucléotide 24—1166

| | | | | | | | | | | | | |
|---|---|---|---|---|---|---|---|---|---|---|---|---|
| ATG | AGG | TGT | CTC | GTG | GTG | CTA | CTT | GCT | GTC | TTC | GCT | CTC |
| 24 | | | | | | | | | | | | |
| TCC | CAA | GGC | GCT | GAG | ATC | ACC | AGG | ATC | CCT | CTG | TAC | AAA |
| 63 | | | 72 | | | | | | | | | |
| GGC | AAG | TCT | CTG | AGG | AAG | GCG | CTG | AAG | GAG | CAT | GGG | CTT |
| 102 | | | | | | | 123 | | | | | |
| CTG | GAG | GAC | TTC | CTG | CAG | AAA | CAG | CAG | TAT | GGC | ATC | AGC |
| 141 | | | | 153 | | | | | | | | |
| AGC | AAG | TAC | TCC | GGC | TTC | GGG | GAG | GTG | GCC | AGC | GTG | CCC |
| 180 | 183 | | | | | 198 | | | | | | |
| CTG | ACC | AAC | TAC | CTG | GAT | AGT | CAG | TAC | TTT | GGG | AAG | ATC |
| 219 | | | | | | | | 243 | | | | |
| TAC | CTC | GGG | ACC | CCG | CCC | CAG | GAG | TTC | ACC | GTG | CTG | TTT |
| 258 | | | | | | | | | | | | |
| GAC | ACT | GGC | TCC | TCT | GAC | TTC | TGG | GTA | CCC | TCT | ATC | TAC |
| 297 | | 303 | | | | | | | | | | |
| TGC | AAG | AGC | AAT | GCC | TGC | AAA | AAC | CAC | CAG | CGC | TTC | GAC |
| 336 | | | | | | | | | 363 | | | |
| CCG | AGA | AAG | TCG | TCC | ACC | TTC | CAG | AAC | CTG | GGC | AAG | CCC |
| 375 | | | | | | | | | | | | |
| CTG | TCT | ATC | CAC | TAC | GGG | ACA | GGC | AGC | ATG | CAG | GGC | ATC |
| 414 | | | 423 | | | | | | | | | |
| CTG | GGC | TAT | GAC | ACC | GTC | ACT | GTC | TCC | AAC | ATT | GTG | GAC |
| 453 | | | | | | | | | | 483 | | |
| ATC | CAG | CAG | ACA | GTA | GGC | CTG | AGC | ACC | CAG | GAG | CCC | GGG |
| 492 | | | | | | | | | | | | |
| GAC | GTC | TTC | ACC | TAT | GCC | GAA | TTC | GAC | GGG | ATC | CTG | GGG |
| 531 | | | | 543 | | | | | | | | |

ATG GCC TAC CCC TCG CTC GCC TCA GAG TAC TCG ATA CCC
570 ... 603

GTG TTT GAC AAC ATG ATG AAC AGG CAC CTG GTG GCC CAA
609

GAC CTG TTC TCG GTT TAC ATG GAC AGG AAT GGC CAG GAG
648 ... 663 ... 675

AGC ATG CTC ACG CTG GGG GCC ATC GAC CCG TCC TAC TAC
687 ... 723

ACA GGG TCC CTG CAT TGG GTG CCC GTG ACA GTG CAG CAG
726

TAC TGG CAG TTC ACT GTG GAC AGT GTC ACC ATC AGC GGT
765 ... 783

GTG GTT GTG GCC TGT GAG GGT GGC TCT CAG GCC ATC CTG
804

GAC ACG GGC ACC TCC AAG CTG GTC GGG CCC AGC AGC GAC
843

ATC CTC AAC ATC CAG CAG GCC ATT GGA GCC ACA CAG AAC
882 ... 903

CAG TAC GGT GAG TTT GAC ATC GAC TGC GAC AAC CTG AGC
921 ... 928

TAC ATG CCC ACT GTG GTC TTT GAG ATC AAT GGC AAA ATG
960

TAC CCA CTG ACC CCC TCC GCC TAT ACC AGC CAG GAC CAG
999 ... 1023

GGC TTC TGT ACC AGT GCC TTC CAG AGT GAA AAT CAT TCC
1038

CAG AAA TGG ATC CTG GGG GAT GTT TTC ATC CGA GAG TAT
1077 ... 1083

TAC AGC GTC TTT GAC AGG GCC AAC AAC CTC GTG GGG CTG
1116 ... 1143

GCC AAA GCC ATC,
1155 ... 1166

(b) le polynucléotide 24—1166 qui est le même que celui de (a), si ce n'est que le nucléotide A en position 675 est remplacé par G,

(c) le polynucléotide 24—1166 qui est le même que celui de (a), si ce n'est que le nucléotide G en position 928 est remplacé par A, et

(d) le polynucléotide 24—1166 qui est le même que celui de (a), si ce n'est que le nucléotide A en position 675 est remplacé par G et le nucléotide G en position 928 est remplacé par A.

3. Plasmide recombinant selon la Revendication 1, dans lequel le brin plus de l'ADNr-ds de la prochymosine de (1) est choisi à partir des séquences suivantes

(a) le polynucléotide 72—1166 indiqué dans la Revendication 2(a),

(b) le polynucléotide 72—1166 indiqué dans la Revendication 2(b),

(c) le polynucléotide 72—1166 indiqué dans la Revendication 2(c) et

(d) le polynucléotide 72—1166 indiqué dans la Revendication 2(d).

4. Plasmide recombinant selon la Revendication 1, dans lequel le brin plus de l'ADNr-ds de la pseudochymosine de (1) est choisi à partir des séquences suivantes

(a) le polynucléotide 153—1166 indiqué dans la Revendication 2(a),

(b) le polynucléotide 153—1166 indiqué dans la Revendication 2(b),

(c) le polynucléotide 153—1166 indiqué dans la Revendication 2(c) et

(d) le polynucléotide 153—1166 indiqué dans la Revendication 2(d).

5. Plasmide recombinant selon la Revendication 1, dans lequel le brin plus de l'ADNr-ds de la chymosine de (1) est choisi à partir des séquences suivantes

(a) le polynucléotide 198—1166 indiqué dans la Revendication 2(a),

(b) le polynucléotide 198—1166 indiqué dans la Revendication 2(b),

(c) le polynucléotide 198—1166 indiqué dans la Revendication 2(c) et

(d) le polynucléotide 198—1166 indiqué dans la Revendication 2(d).

6. Plasmide recombinant selon la Revendication 1, comprenant un régulon consistant en un système double lac UV5.

7. Plasmide recombinant selon la Revendication 1, comprenant un régulon consistant en au moins un système tryptophane modifié, dans lequel l'information codant pour la protéine de l'atténuateur trp est éliminée.

8. Plasmide recombinant selon la Revendication 7, comprenant deux régulons trp modifiés liés selon le mode tête à queue.

9. Plasmide recombinant selon la Revendication 1, comprenant un réglon consistant au moins en un site de liaison ribosome/promoteur modifié du gène VIII du bactériophage M13.

10. Plasmide recombinant selon la Revendication 1, comprenant un mutant de réplication thermosensible dérivé du plasmide pVU 208 de la cloacine DF 13.

11. Plasmide recombinant selon la Revendication 1, choisi à partir de pUR 1521 (ATCC 39120), pUR 1533 (ATCC 39121), pUR 1734 (ATCC 39198) et pUR 1832 (ATCC 39197).

12. Culture bactérienne comprenant des cellules d'*E. coli* transformées avec des plasmides selon l'une quelconque des Revendications 1 à 11.

13. Procédé de production de la préprochymosine ou de l'une quelconque de ses formes de maturation, prochymosine, pseudochymosine ou chymosine, qui comprend la culture d'une culture bactérienne selon la Revendication 12 sous une pression de sélection et la récupération de la préprochymosine ou d'une forme de maturation de cette dernière.

14. Microorganismes qui sont transformés par l'incorportion

(a) d'un ADNr-ds codant pour la préprochymosine, la prochymosine, la pseudochymosine de la chymosine,

(b) d'un codon de terminaison de traduction lié à l'extrémité 3' du brin plus de l'ADNr-ds de (a),

(c) d'un marqueur séléctif et de préférence d'un site de réplication adapté auxdits microorganismes,

(d) d'un régulon d'expression approprié pour lesdits microorganisme en amont du brin plus de l'ADNr-ds de (a), et

(e) lorsque l'ADNr-ds code pour la prochymosine, la pseudochymosine ou la chymosine, d'un triplet ATG d'initiation de traduction lié à l'extrémité 5' du brin plus de l'ADNr-ds de (a),

les éléments a, b, d et éventuellement e étant présents dans l'ordre d-(e)-a-b.

15. Microorganismes selon la Revendication 14, où l'ADNr-ds est choisi parmi les séquences d'ADNr-ds spécifiées dans les Revendications 2(a)—(d), 3(a)—(d), 4(a)—(d) et 5(a)—(d).

16. Microorganismes selon la Revendication 14 ou 15, où l'ADNr-ds code pour la préprochymosine.

17. Microorganismes selon l'une quelconque des Revendications 14 à 16, où le microorganisme est non toxique et comestible et est choisi dans le groupe consistant en streptocoques ou lactobacilles ou microorganismes du genre *Bacillus* ou de levures.

18. Procédé de production de la préprochymosine ou de l'une quelconque de ses formes de maturation prochymosine, pseudochymosine ou chymosine, qui comprend la culture d'un microorganisme selon l'une quelconque des Revendications 14 à 17, éventuellement sous une pression de sélection et le recueil de la préprochymosine ou d'une forme de maturation de cette dernière.

**Revendications pour l'Etat Contractant: Au**

1. Procédé de préparation d'un plasmide recombinant qui comprend la combinaison, par des techniques de recombinaison de l'ADN, des éléments suivants dans l'ordre indiqué

(1) un ADNr-ds codant pour la préprochymosine, la prochymosine, la pseudochymosine ou la chymosine,

(2) un codon de terminaison de traduction lié à l'extrémité 3' du brin plus de l'ADNr-ds de (1),

(3) à la fois un site de réplication d'*E. coli* et un marqueur sélectif,

(4) un régulon d'expression d'*E. coli* en amont du brin plus de l'ADNr-ds de (1), et

(5) lorsque l'ADNr-ds code pour la prochymosine, la pseudochymosine ou la chymosine, un triplet ATG d'initiation de traduction lié à l'extrémité 5' du brin plus de l'ADNr-ds de (1).

2. Procédé selon la Revendication 1, dans lequel le brin plus de l'ADNr-ds de la préprochymosine de (1) est choisi à partir des séquences suivantes

(a) le polynucléotide 24—1166

| | | | | | | | | | | | | |
|---|---|---|---|---|---|---|---|---|---|---|---|---|
| ATG | AGG | TGT | CTC | GTG | GTG | CTA | CTT | GCT | GTC | TTC | GCT | CTC |
| 24 | | | | | | | | | | | | |
| TCC | CAA | GGC | GCT | GAG | ATC | ACC | AGG | ATC | CCT | CTG | TAC | AAA |
| 63 | | | 72 | | | | | | | | | |
| GGC | AAG | TCT | CTG | AGG | AAG | GCG | CTG | AAG | GAG | CAT | GGG | CTT |
| 102 | | | | | | | 123 | | | | | |
| CTG | GAG | GAC | TTC | CTG | CAG | AAA | CAG | CAG | TAT | GGC | ATC | AGC |
| 141 | | | | 153 | | | | | | | | |
| AGC | AAG | TAC | TCC | GGC | TTC | GGG | GAG | GTG | GCC | AGC | GTG | CCC |
| 180 | 183 | | | | | 198 | | | | | | |
| CTG | ACC | AAC | TAC | CTG | GAT | AGT | CAG | TAC | TTT | GGG | AAG | ATC |
| 219 | | | | | | | | 243 | | | | |
| TAC | CTC | GGG | ACC | CCG | CCC | CAG | GAG | TTC | ACC | GTG | CTG | TTT |
| 258 | | | | | | | | | | | | |
| GAC | ACT | GGC | TCC | TCT | GAC | TTC | TGG | GTA | CCC | TCT | ATC | TAC |
| 297 | | 303 | | | | | | | | | | |
| TGC | AAG | AGC | AAT | GCC | TGC | AAA | AAC | CAC | CAG | CGC | TTC | GAC |
| 336 | | | | | | | | | 363 | | | |
| CCG | AGA | AAG | TCG | TCC | ACC | TTC | CAG | AAC | CTG | GGC | AAG | CCC |
| 375 | | | | | | | | | | | | |
| CTG | TCT | ATC | CAC | TAC | GGG | ACA | GGC | AGC | ATG | CAG | GGC | ATC |
| 414 | | | 423 | | | | | | | | | |
| CTG | GGC | TAT | GAC | ACC | GTC | ACT | GTC | TCC | AAC | ATT | GTG | GAC |
| 453 | | | | | | | | | | 483 | | |
| ATC | CAG | CAG | ACA | GTA | GGC | CTG | AGC | ACC | CAG | GAG | CCC | GGG |
| 492 | | | | | | | | | | | | |

GAC GTC TTC ACC TAT GCC GAA TTC GAC GGG ATC CTG GGG
531 543

ATG GCC TAC CCC TCG CTC GCC TCA GAG TAC TCG ATA CCC
570 603

GTG TTT GAC AAC ATG ATG AAC AGG CAC CTG GTG GCC CAA
609

GAC CTG TTC TCG GTT TAC ATG GAC AGG AAT GGC CAG GAG
648 663 675

AGC ATG CTC ACG CTG GGG GCC ATC GAC CCG TCC TAC TAC
687 723

ACA GGG TCC CTG CAT TGG GTG CCC GTG ACA GTG CAG CAG
726

TAC TGG CAG TTC ACT GTG GAC AGT GTC ACC ATC AGC GGT
765 783

GTG GTT GTG GCC TGT GAG GGT GGC TCT CAG GCC ATC CTG
804

GAC ACG GGC ACC TCC AAG CTG GTC GGG CCC AGC AGC GAC
843

ATC CTC AAC ATC CAG CAG GCC ATT GGA GCC ACA CAG AAC
882 903

CAG TAC GGT GAG TTT GAC ATC GAC TGC GAC AAC CTG AGC
921 928

TAC ATG CCC ACT GTG GTC TTT GAG ATC AAT GGC AAA ATG
960

TAC CCA CTG ACC CCC TCC GCC TAT ACC AGC CAG GAC CAG
999 1023

GGC TTC TGT ACC AGT GCC TTC CAG AGT GAA AAT CAT TCC
1038

CAG AAA TGG ATC CTG GGG GAT GTT TTC ATC CGA GAG TAT
1077 1083

TAC AGC GTC TTT GAC AGG GCC AAC AAC CTC GTG GGG CTG
1116 1143

GCC AAA GCC ATC,
1155 1166

(b) le polynucléotide 24—1166, qui est le même que celui de (a), si ce n'est que le nucléotide A en position 675 est remplacé par G,

(c) le polynucléotide 24—1166, qui est le même que celui de (a), si ce n'est que le nucléotide G en position 928 est remplacé par A, et

(d) le polynucléotide 24—1166, qui est le même que celui de (a), si ce n'est que le nucléotide A en position 675 est remplacé par G et le nucléotide G en position 928 est remplacé par A.

3. Procédé selon la Revendication 1, dans lequel le brin plus de l'ADNr-ds de la prochymosine de (1) est choisi à partir des séquences suivantes

(a) le polynucléotide 72—1166 indiqué dans la Revendication 2(a),

(b) le polynucléotide 72—1166 indiqué dans la Revendication 2(b),

(c) le polynucléotide 72—1166 indiqué dans la Revendication 2(c), et

(d) le polynucléotide 72—1166 indiqué dans la Revendication 2(d).

4. Procédé selon la Revendication 1, dans lequel le brin plus de l'ADNr-ds la pseudochymosine de (1) est choisi à partir des séquences suivantes

(a) le polynucléotide 153—1166 indiqué dans la Revendication 2(a),

(b) le polynucléotide 153—1166 indiqué dans la Revendication 2(b),

(c) le polynucléotide 153—1166 indiqué dans la Revendication 2(c), et

(d) le polynucléotide 153—1166 indiqué dans la Revendication 2(d).

5. Procédé selon la Revendication 1, dans lequel le brin plus de l'ADNr-ds de la chymosine de (1) est choisi à partir des séquences suivantes

(a) le polynucléotide 198—1166 indiqué dans la Revendication 2(a),

(b) le polynucléotide 198—1166 indiqué dans la Revendication 2(b),

(c) le polynucléotide 198—1166 indiqué dans la Revendication 2(c), et

(d) le polynucléotide 198—1166 indiqué dans la Revendication 2(d).

6. Procédé selon la Revendication 1, dans lequel le régulon de (4) est un système double lac UV5.

7. Procédé selon la revendication 1, dans lequel le régulon de (4) est au moins un système tryptophane modifié, dans lequel l'information codant pour la protéine de l'atténuateur trp est éliminée.

8. Procédé selon la Revendication 7, dans lequel deux régulons trp modifiés sont liés selon un mode tête à queue.

9. Procédé selon la Revendication 1, dans lequel le régulon de (4) est au moins un site de liaison ribsome/promoteur modifié du gène VIII du bactériophage M13.

10. Procédé selon la Revendication 1, dans lequel le plasmide comprend un mutant de réplication thermosensible dérivé du plasmide pVU 208, de la cloacine DF 13.

11. Procédé selon la Revendication 1, dans lequel le plasmide recombinant est choisi à partir de pUR 1521 (ATCC 39120), pUR 1533 (ATCC 39121), pUR 1734 (ATCC 39198) et pUR 1832 (ATCC 39197).

12. Procédé de préparation d'une culture bactérienne qui comprend la transformation de cellules d'*E. coli* avec des plasmides préparés par un procédé tel qu'il est décrit dans l'une quelconque des Revendications 1 à 11.

13. Procédé de production de la préprochymosine ou de l'une quelconque de ses formés de maturation prochymosine, pseudochymosine ou chymosine, qui comprend la culture d'une culture bactérienne préparée par un procédé tel qu'il est décrit dans la Revendication 12 sous une pression de sélection et la récupération de la préprochymosine ou d'une forme de maturation de cette dernière.

14. Procédé de préparation de microorganismes transformés, qui comprend l'incorporation dans les microorganismes

(a) d'un ADNr-ds codant pour la préprochymosine, la prochymosine, la pseudochymosine ou chymosine,

(b) d'un codon de terminaison de traduction lié à l'extrémité 3' du brin plus de l'ADNr-ds de (a),

(c) d'un marqueur sélectif et de préférence d'un site de réplication adapté auxdits microorganismes,

(d) d'un régulon d'expression approprié pour lesdits microorganismes en amont du brin plus de l'ADNr-ds de (a), et

(e) lorsque l'ADNr-ds code pour la prochymosine, la pseudochymosine ou la chymosine, d'un triplet ATG d'initiation de traduction lié à l'extrémité 5' du brin plus de l'ADNr-ds de (a),

les éléments a, b, d et éventuellement e étant arrangés dans l'ordre d-(e)-a-b.

15. Procédé selon la Revendication 14, dans lequel l'ADNr-ds est choisi à partir des séquences d'ADNr-ds spécifiées dans les Revendications 2(a)—(d), 3(a)—(d), 4(a)—(d) et 5(a)—(d).

16. Procédé selon la Revendication 14 ou 15, dans lequel l'ADNr-ds code pour la préprochymosine.

17. Procédé selon l'une quelconque des Revendications 14 à 16, dans lequel le microorganisme est non toxique et comestible et est choisi dans le groupe consistant en streptocoques ou lactobacilles ou microorganismes du genre *Bacillus* ou de levures.

18. Procédé de production de la préprochymosine ou de l'une quelconque de ses formes de maturation prochymosine, pseudochymosine ou chymosine, qui comprend la culture d'un microorganisme préparé par un procédé tel qu'il est décrit dans l'une quelconque des Revendications 14 à 17, éventuellement sous une pression de sélection et la récupération de la préprochymosine ou d'une forme de maturation de cette dernière.

```
                                         s1                  s5                      s10
                                         met arg cys leu val val leu leu ala val phe ala leu
CAGCG GCT GGA CCC AGA TCC AAG ATG AGG TGT CTC GTG GTG CTA CTT GCT GTC TTC GCT CTC
  3

     s15     1                                           10
 ser gln gly ALA GLU ILE THR ARG ILE PRO LEU TYR LYS GLY LYS SER LEU ARG LYS ALA
 TCC CAA GGC GCT GAG ATC ACC AGG ATC CCT CTG TAC AAA GGC AAG TCT CTG AGG AAC GCG
 63

         20                                              30
 LEU LYS GLU HIS GLY LEU LEU GLU ASP PHE LEU GLN LYS GLN GLN TYR GLY ILE SER SER
 CTG AAG GAG CAT GGG CTT CTG GAG GAC TTC CTG CAG AAA CAG CAG TAT GGC ATC AGC AGC
 123

         40                                              50
 LYS TYR SER GLY PHE GLY GLU VAL ALA SER VAL PRO LEU THR ASN TYR LEU ASP SER GLN
 AAG TAC TCC GGC TTC GGG GAG GTG GCC AGC GTG CCC CTG ACC AAC TAC CTG GAT AGT CAG
 183

         60                                              70
 TYR PHE GLY LYS ILE TYR LEU GLY THR PRO PRO GLN GLU PHE THR VAL LEU PHE ASP THR
 TAC TTT GGG AAG ATC TAC CTC GGG ACC CCG CCC CAG GAG TTC ACC GTG CTG TTT GAC ACT
 243

         80                                              90
 GLY SER SER ASP PHE TRP VAL PRO SER ILE TYR CYS LYS SER ASN ALA CYS LYS ASN HIS
 GGC TCC TCT GAC TTC TGG GTA CCC TCT ATC TAC TGC AAG AGC AAT GCC TGC AAA AAC CAC
 303

         100                                             110
 GLN ARG PHE ASP PRO ARG LYS SER SER THR PHE GLN ASN LEU GLY LYS PRO LEU SER ILE
 CAG CGC TTC GAC CCG AGA AAG TCG TCC ACC TTC CAG AAC CTG GGC AAG CCC CTG TCT ATC
 363

         120                                             130
 HIS TYR GLY THR GLY SER MET GLN GLY ILE LEU GLY TYR ASP THR VAL THR VAL SER ASN
 CAC TAC GGG ACA GGC AGC ATG CAG GGC ATC CTG GGC TAT GAC ACC GTC ACT GTC TCC AAC
 423
         140                                             150
 ILE VAL ASP ILE GLN GLN THR VAL GLY LEU SER THR GLN GLU PRO GLY ASP VAL PHE THR
 ATT GTG GAC ATC CAG CAG ACA GTA GGC CTG AGC ACC CAG GAG CCC GGG GAC GTC TTC ACC
 483

         160                                             170
 TYR ALA GLU PHE ASP GLY ILE LEU GLY MET ALA TYR PRO SER LEU ALA SER GLU TYR SER
 TAT GCC GAA TTC GAC GGG ATC CTG GGG ATG GCC TAC CCC TCG CTC GCC TCA CAG TAC TCG
 543

         180                                             190
 ILE PRO VAL PHE ASP ASN MET MET ASN ARG HIS LEU VAL ALA GLN ASP LEU PHE SER VAL
 ATA CCC GTG TTT GAC AAC ATG ATG AAC AGG CAC CTG GTG GCC CAA GAC CTG TTC TCG GTT
 603

         200                                             210
 TYR MET ASP ARG ASN GLY GLN GLU SER MET LEU THR LEU GLY ALA ILE ASP PRO SER TYR
 TAC ATG GAC AGG AAT GGC CAG GAG AGC ATG CTC ACC CTG GGG GCC ATC GAC CCG TCC TAC
 663
```

Fig. 1

```
        220                                     230
TYR THR GLY SER LEU HIS TRP VAL PRO VAL THR VAL GLN GLN TYR TRP GLN PHE THR VAL
TAC ACA GGG TCC CTG CAT TGG GTG CCC GTC ACA GTG CAG CAG TAC TGG CAG TTC ACT GTG
723

        240                                     250
ASP SER VAL THR ILE SER GLY VAL VAL VAL ALA CYS GLU GLY GLY CYS GLN ALA ILE LEU
GAC AGT GTC ACC ATC AGC GGT GTG GTT GTG GCC TGT GAG GGT GGC TGT CAG GCC ATC CTG
783

        260                                     270
ASP THR GLY THR SER LYS LEU VAL GLY PRO SER SER ASP ILE LEU ASN ILE GLN GLN ALA
GAC ACG GGC ACC TCC AAG CTG GTC GGG CCC AGC AGC GAC ATC CTC AAC ATC CAG CAG GCC
843

        280                                     290
ILE GLY ALA THR GLN ASN GLN TYR GLY GLU PHE ASP ILE ASP CYS ASP ASN LEU SER TYR
ATT GGA GCC ACA CAG AAC CAG TAC GGT GAG TTT GAC ATC GAC TGC GAC AAC CTG AGC TAC
903

        300                                     310
MET PRO THR VAL VAL PHE GLU ILE ASN GLY LYS MET TYR PRO LEU THR PRO SER ALA TYR
ATG CCC ACT GTG GTC TTT GAG ATC AAT GGC AAA ATG TAC CCA CTG ACC CCC TCC GCC TAT

        320                                     330
THR SER GLN ASP GLN GLY PHE CYS THR SER GLY PHE GLN SER GLU ASN HIS SER GLN LYS
ACC AGC CAG GAC CAG GGC TTC TGT ACC AGT GGC TTC CAG AGT GAA AAT CAT TCC CAG AAA
1023

        340                                     350
TRP ILE LEU GLY ASP VAL PHE ILE ARG GLU TYR TYR SER VAL PHE ASP ARG ALA ASN ASN
TGG ATC CTG GGG GAT GTT TTC ATC CGA GAG TAT TAC AGC GTC TTT GAC AGG GCC AAC AAC
1083

        360                 365
LEU VAL GLY LEU ALA LYS ALA ILE ***
CTC GTG GGG CTG GCC AAA GCC ATC TGA TCA CAT CGC TGA CCA AGA ACC TCA CTG TCC CCA
1143

CAC ACC TGC ACA CAC ACA TGC ACA CAT GTA CAT GAC CAC ATG TGC ACA CAC ACA GAT GAG
1203

GTT TCC AGA CAG ATG ATT CTC AAT AAA CGT TGT CTT TCT GCA AAA AAA A
1263                                               1303
```

Fig. 1, continued.

Fig.2

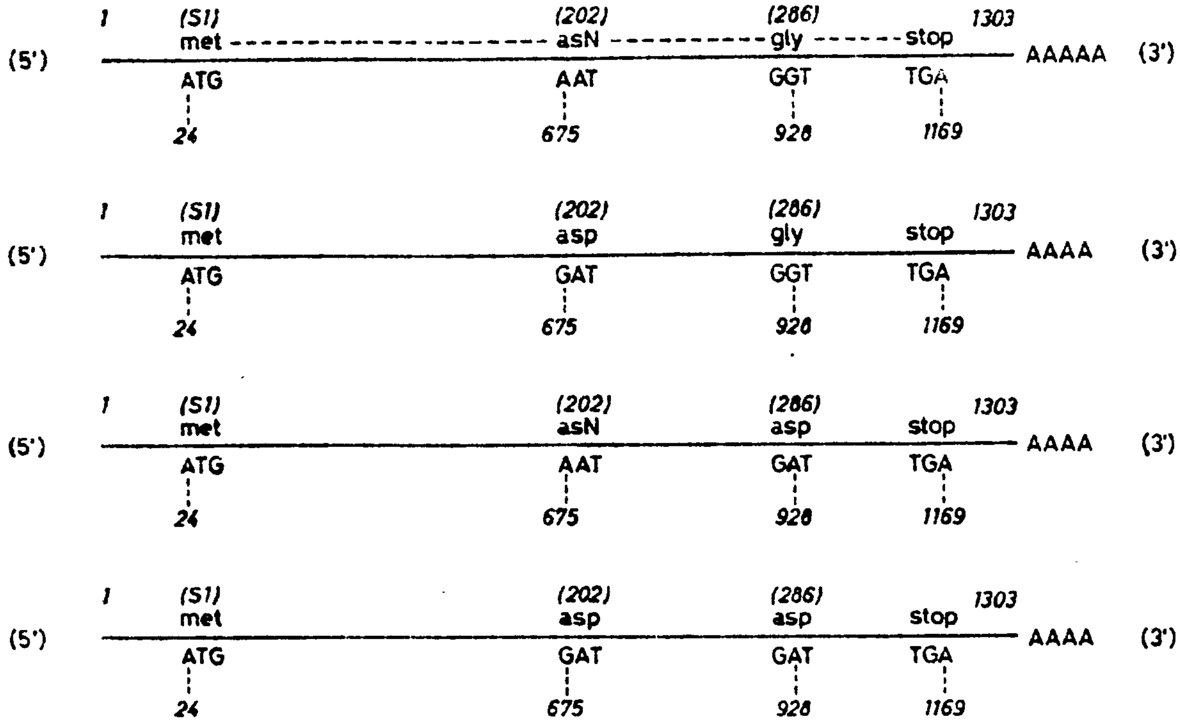
1
(S1)
met
(202)
asN
(286)
gly
stop
1303
(5')
AAAAA
(3')
ATG
AAT
GGT
TGA
24
675
928
1169
1
(S1)
met
(202)
asp
(286)
gly
stop
1303
(5')
AAAA
(3')
ATG
GAT
GGT
TGA
24
675
928
1169
1
(S1)
met
(202)
asN
(286)
asp
stop
1303
(5')
AAAA
(3')
ATG
AAT
GAT
TGA
24
675
928
1169
1
(S1)
met
(202)
asp
(286)
asp
stop
1303
(5')
AAAA
(3')
ATG
GAT
GAT
TGA
24
675
928
1169

Fig. 3

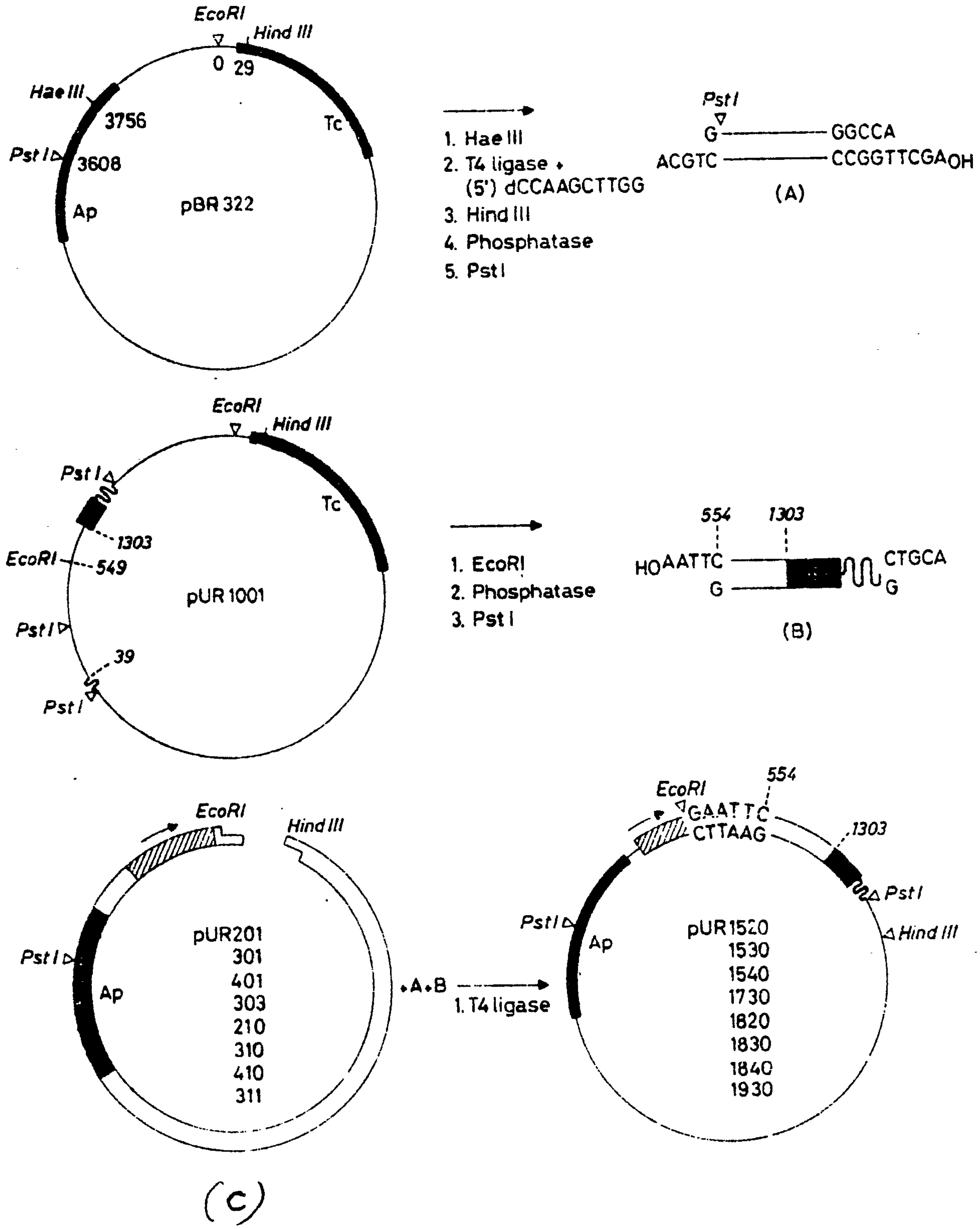
EcoRI
Hind III
0
29
Hae III
3756
Pst I
3608
Tc
Ap
pBR 322
1. Hae III
2. T4 ligase +
(5') dCCAAGCTTGG
3. Hind III
4. Phosphatase
5. Pst I
Pst I
G
GGCCA
ACGTC
CCGGTTCGA OH
(A)
EcoRI
Hind III
Pst I
1303
EcoRI
549
Tc
pUR 1001
Pst I
39
Pst I
1. EcoRI
2. Phosphatase
3. Pst I
554
1303
HO AATTC
G
CTGCA
G
(B)
EcoRI
Hind III
Pst I
Ap
pUR201
301
401
303
210
310
410
311
+A+B
1. T4 ligase
EcoRI
554
GAATTC
CTTAAG
1303
Pst I
Hind III
Pst I
Ap
pUR1520
1530
1540
1730
1820
1830
1840
1930
(C)

Fig. 4

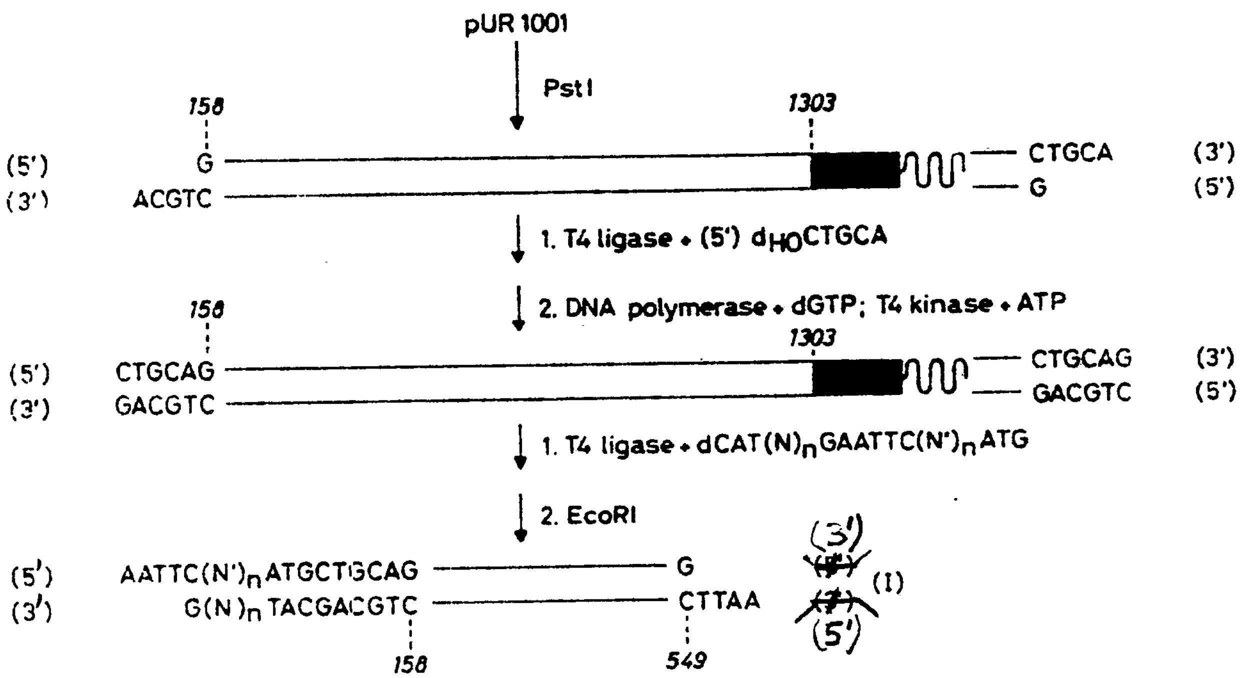
pUR 1001
PstI
158
1303
(5') G
(3') ACGTC
CTGCA (3')
G (5')
1. T4 ligase + (5') dHOCTGCA
2. DNA polymerase + dGTP; T4 kinase + ATP
158
1303
(5') CTGCAG
(3') GACGTC
CTGCAG (3')
GACGTC (5')
1. T4 ligase + dCAT(N)nGAATTC(N')nATG
2. EcoRI
(5') AATTC(N')nATGCTGCAG
(3') G(N)nTACGACGTC
G (3')
CTTAA (5')
(I)
158
549

Fig. 5

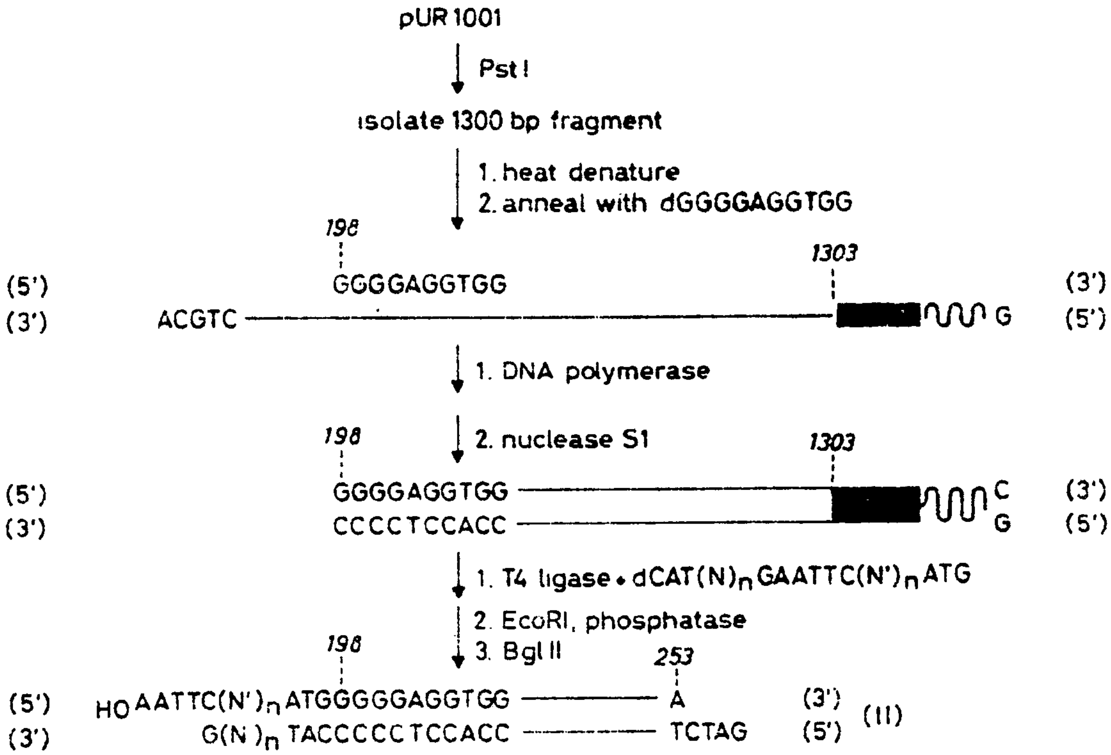
pUR1001
PstI
isolate 1300 bp fragment
1. heat denature
2. anneal with dGGGGAGGTGG
198
1303
(5') GGGGAGGTGG (3')
(3') ACGTC G (5')
1. DNA polymerase
2. nuclease S1
198
1303
(5') GGGGAGGTGG C (3')
(3') CCCCTCCACC G (5')
1. T4 ligase + dCAT(N)n GAATTC(N')n ATG
2. EcoRI, phosphatase
3. BglII
198
253
(5') HO AATTC(N')n ATGGGGGAGGTGG A (3')
(3') G(N)n TACCCCCTCCACC TCTAG (5')
(II)

Fig.6

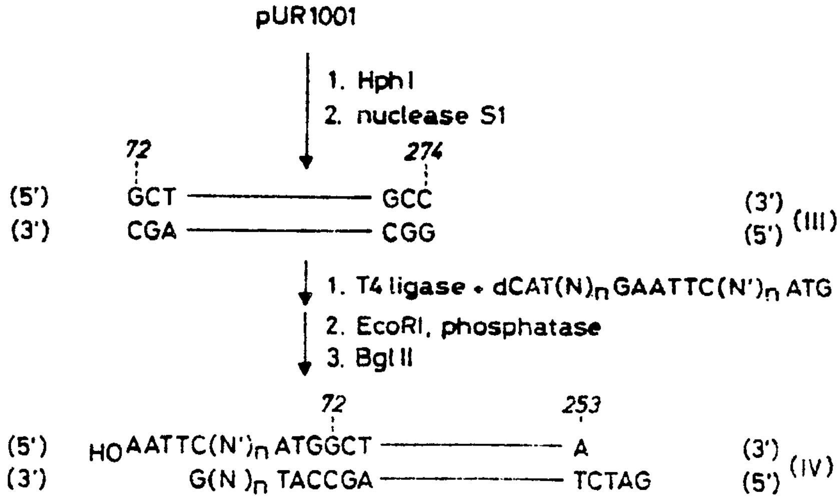
pUR1001
1. HphI
2. nuclease S1
72
274
(5') GCT GCC (3')
(3') CGA CGG (5')
(III)
1. T4 ligase + dCAT(N)n GAATTC(N')n ATG
2. EcoRI, phosphatase
3. BglII
72
253
(5') HO AATTC(N')n ATGGCT A (3')
(3') G(N)n TACCGA TCTAG (5')
(IV)

Fig. 7

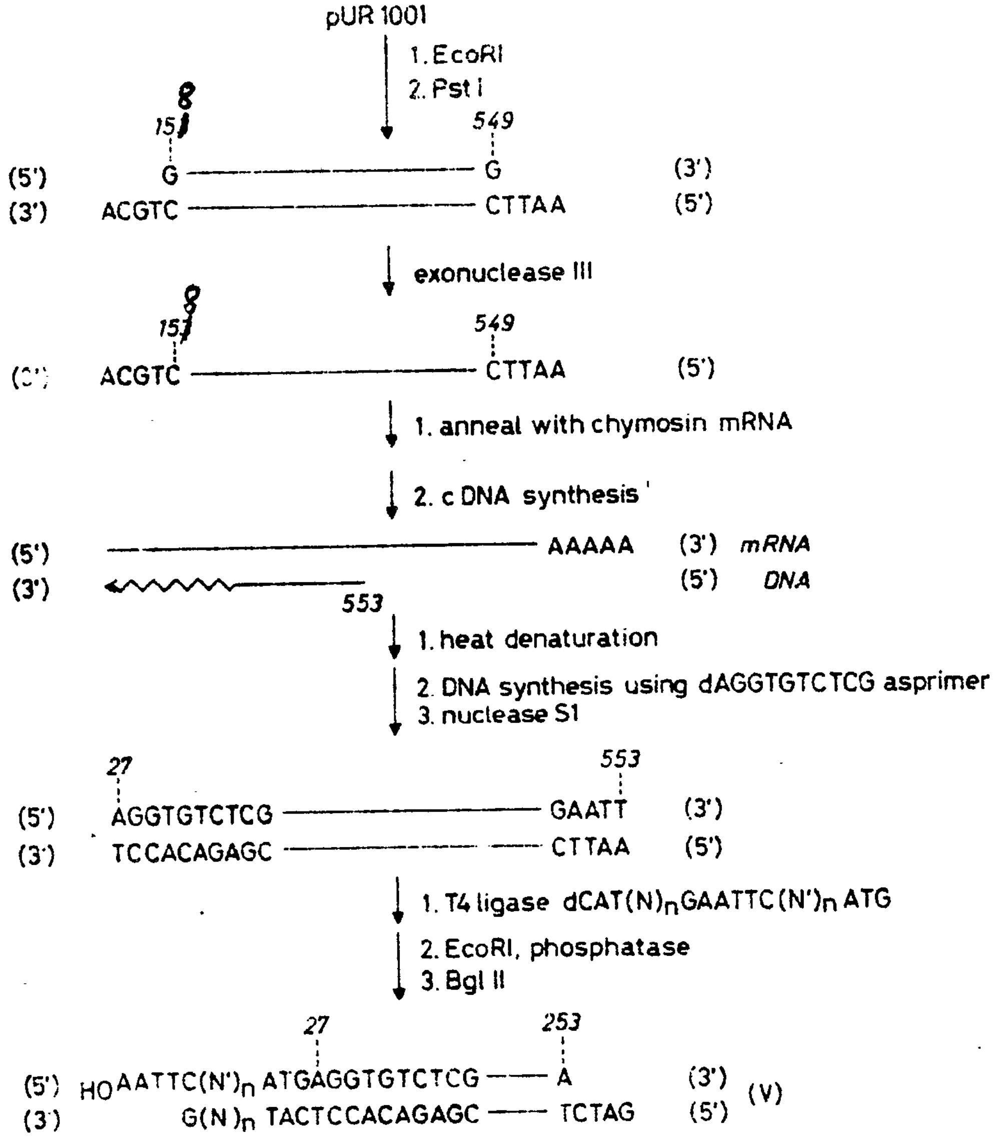
pUR 1001
1. EcoRI
2. Pst I
151
549
(5') G G (3')
(3') ACGTC CTTAA (5')
exonuclease III
151
549
ACGTC CTTAA (5')
1. anneal with chymosin mRNA
2. c DNA synthesis
(5') AAAAA (3') mRNA
(3') (5') DNA
553
1. heat denaturation
2. DNA synthesis using dAGGTGTCTCG asprimer
3. nuclease S1
27
553
(5') AGGTGTCTCG GAATT (3')
(3') TCCACAGAGC CTTAA (5')
1. T4 ligase dCAT(N)nGAATTC(N')n ATG
2. EcoRI, phosphatase
3. Bgl II
27
253
(5') HO AATTC(N')n ATGAGGTGTCTCG A (3')
(3') G(N)n TACTCCACAGAGC TCTAG (5')
(V)

Fig. 8

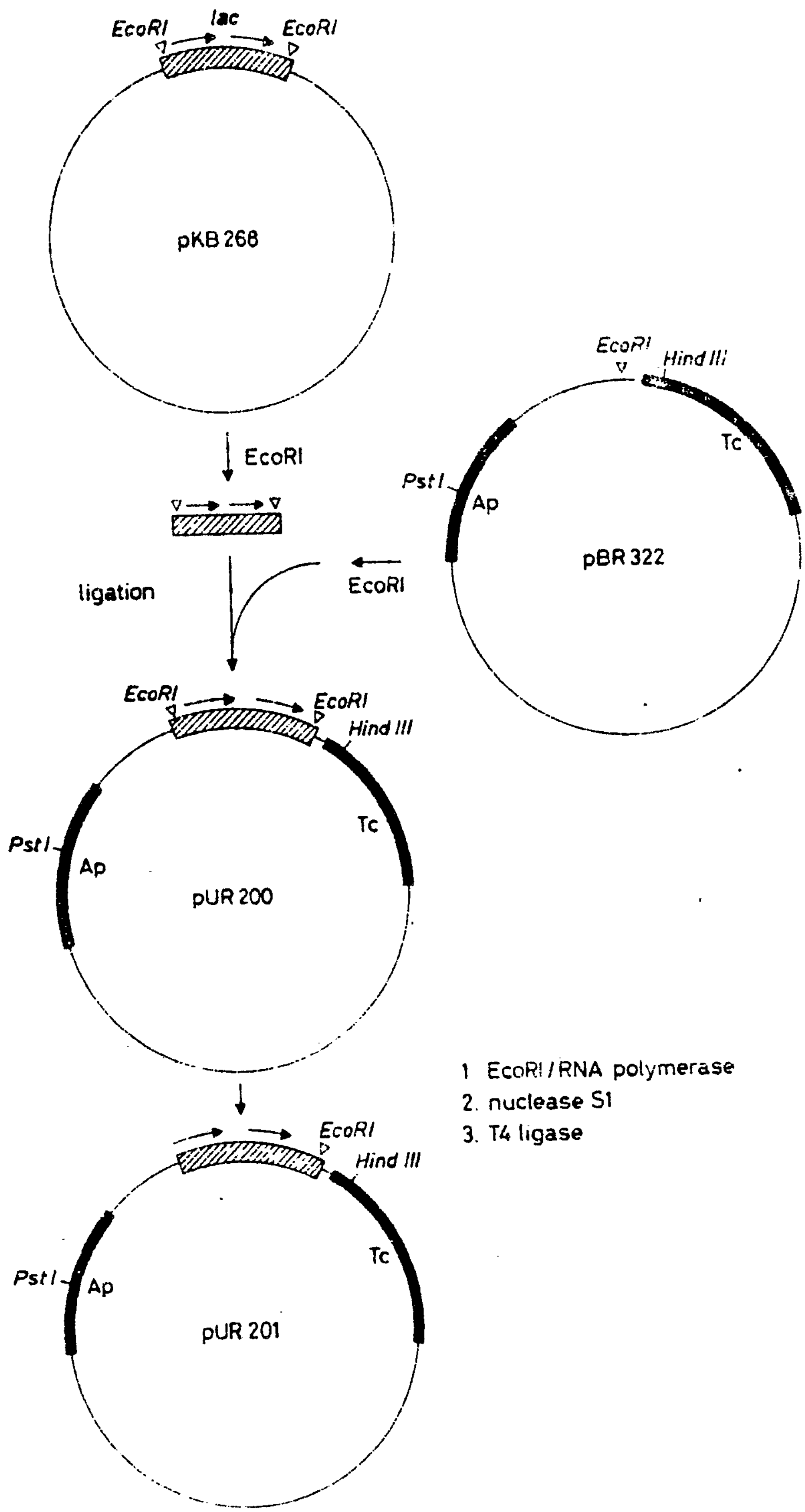
EcoRI
lac
EcoRI
pKB 268
EcoRI
EcoRI
Hind III
Tc
PstI
Ap
pBR 322
ligation
EcoRI
EcoRI
EcoRI
Hind III
Tc
PstI
Ap
pUR 200
1 EcoRI / RNA polymerase
2. nuclease S1
3. T4 ligase
EcoRI
Hind III
Tc
PstI
Ap
pUR 201

Fig. 9

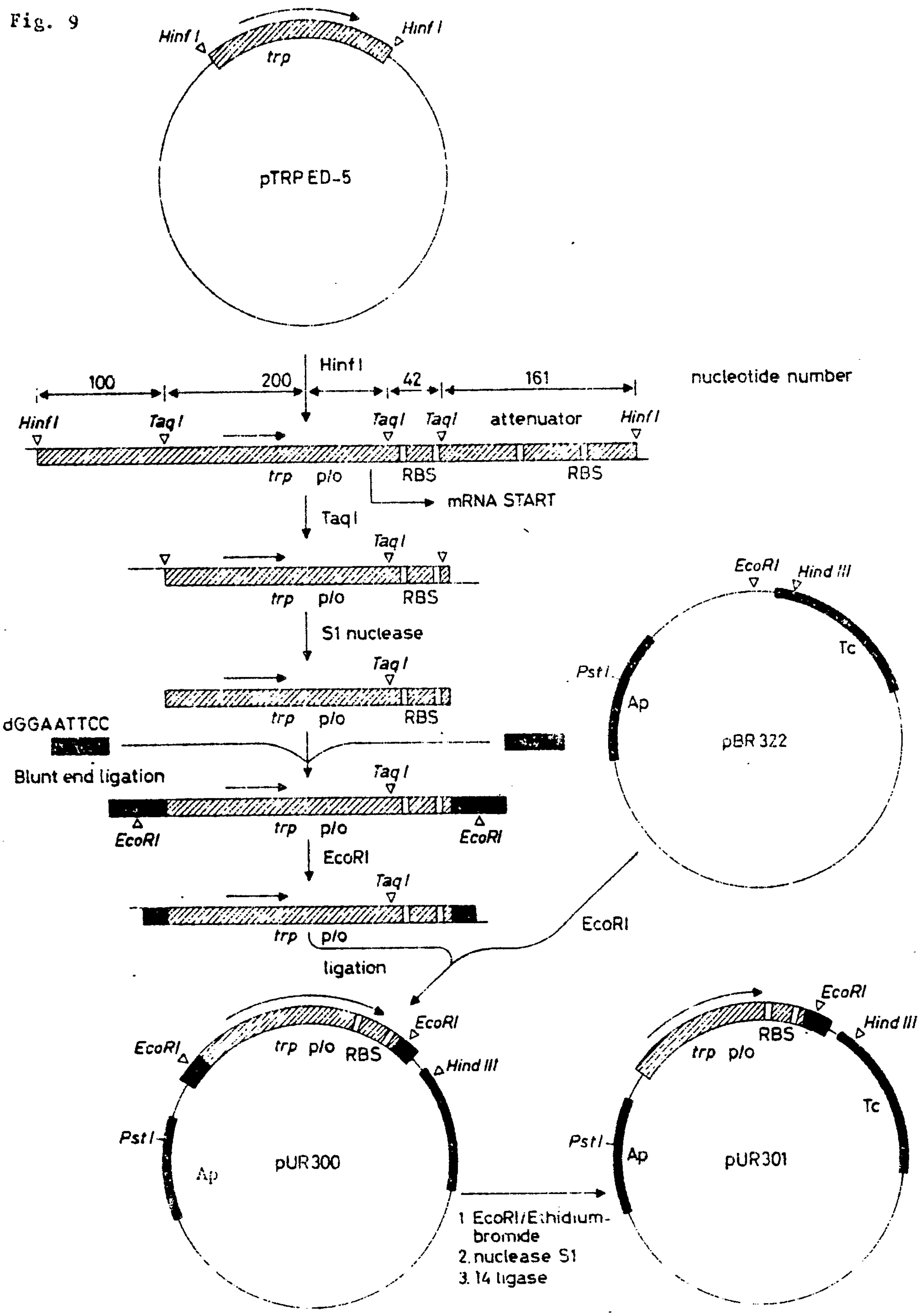
HinfI
HinfI
trp
pTRP ED-5
HinfI
100
200
42
161
nucleotide number
HinfI
TaqI
TaqI
TaqI
attenuator
HinfI
trp p/o
RBS
RBS
mRNA START
TaqI
TaqI
trp p/o
RBS
S1 nuclease
TaqI
trp p/o
RBS
dGGAATTCC
Blunt end ligation
TaqI
EcoRI
trp p/o
EcoRI
EcoRI
TaqI
trp p/o
ligation
EcoRI
EcoRI
HindIII
PstI
Ap
Tc
pBR 322
EcoRI
EcoRI
trp p/o RBS
HindIII
PstI
Ap
pUR300
1 EcoRI/Ethidium-bromide
2. nuclease S1
3. T4 ligase
EcoRI
HindIII
RBS
trp p/o
Tc
PstI
Ap
pUR301

Fig. 10

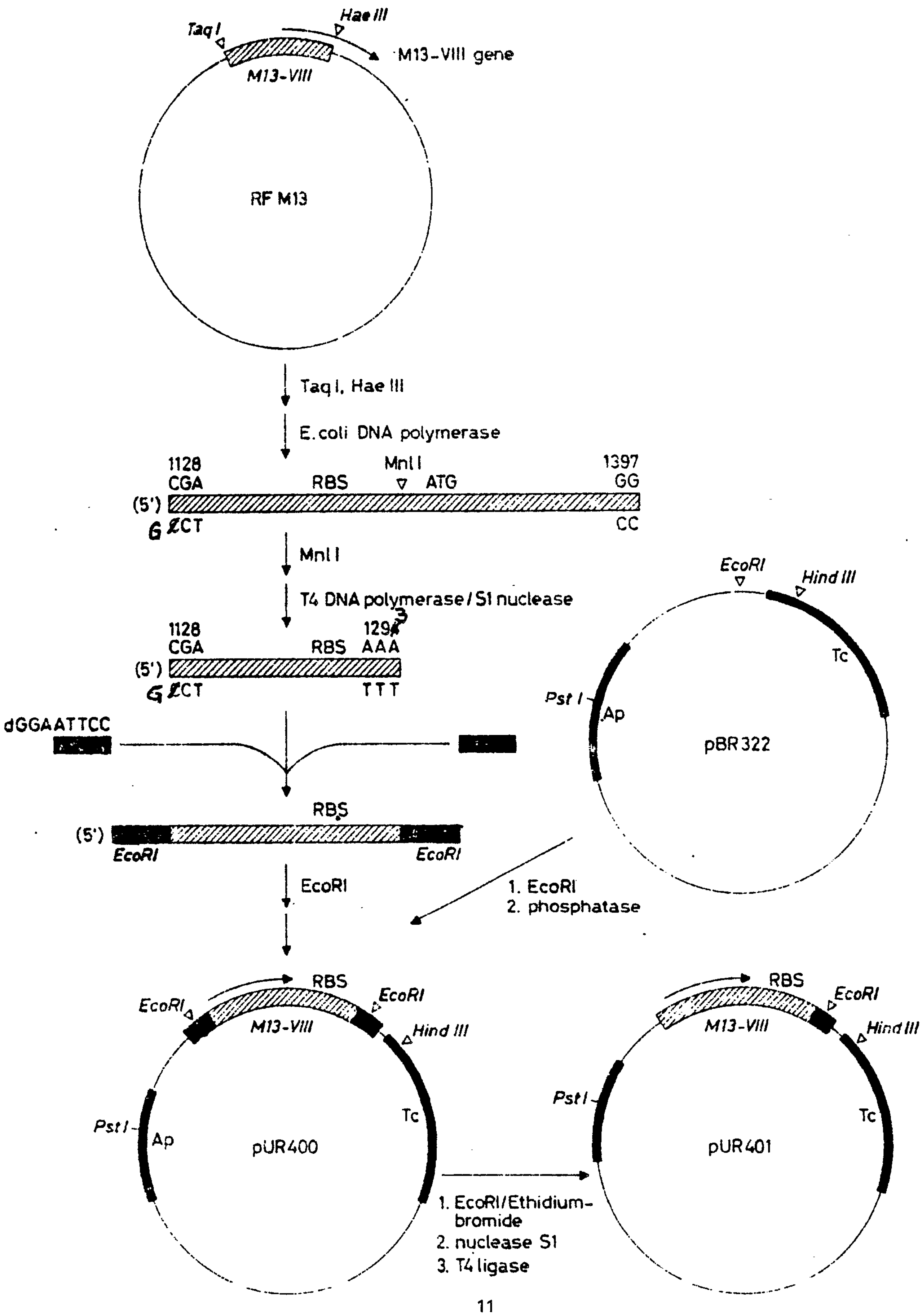
Taq I
Hae III
M13-VIII gene
M13-VIII
RF M13
Taq I, Hae III
E.coli DNA polymerase
1128
CGA
RBS
MnlI
ATG
1397
GG
(5')
GCT
CC
MnlI
T4 DNA polymerase / S1 nuclease
1128
CGA
RBS
1293
AAA
(5')
GCT
TTT
dGGAATTCC
RBS
(5')
EcoRI
EcoRI
EcoRI
EcoRI
HindIII
Tc
PstI
Ap
pBR322
1. EcoRI
2. phosphatase
RBS
EcoRI
EcoRI
M13-VIII
Hind III
PstI
Ap
Tc
pUR400
1. EcoRI/Ethidium-bromide
2. nuclease S1
3. T4 ligase
RBS
EcoRI
M13-VIII
Hind III
PstI
Tc
pUR401

Fig. 11

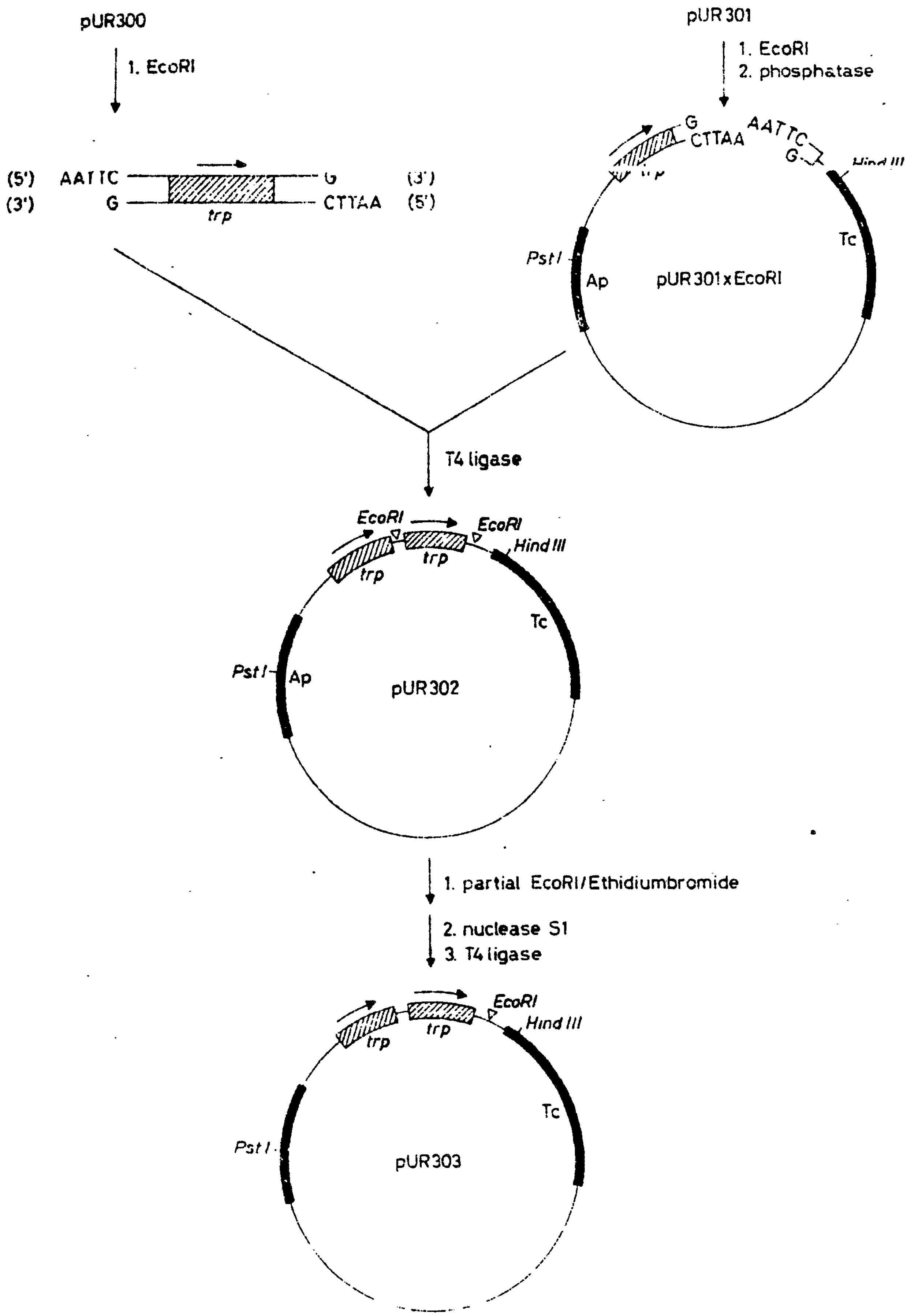
pUR300
1. EcoRI
(5') AATTC
G (3')
(3') G
CTTAA (5')
trp
pUR301
1. EcoRI
2. phosphatase
G
CTTAA
AATTC
G
Hind III
Tc
PstI
Ap
pUR301 x EcoRI
T4 ligase
EcoRI
EcoRI
Hind III
trp
trp
Tc
PstI
Ap
pUR302
1. partial EcoRI/Ethidiumbromide
2. nuclease S1
3. T4 ligase
EcoRI
Hind III
trp
trp
Tc
PstI
pUR303

Fig. 12

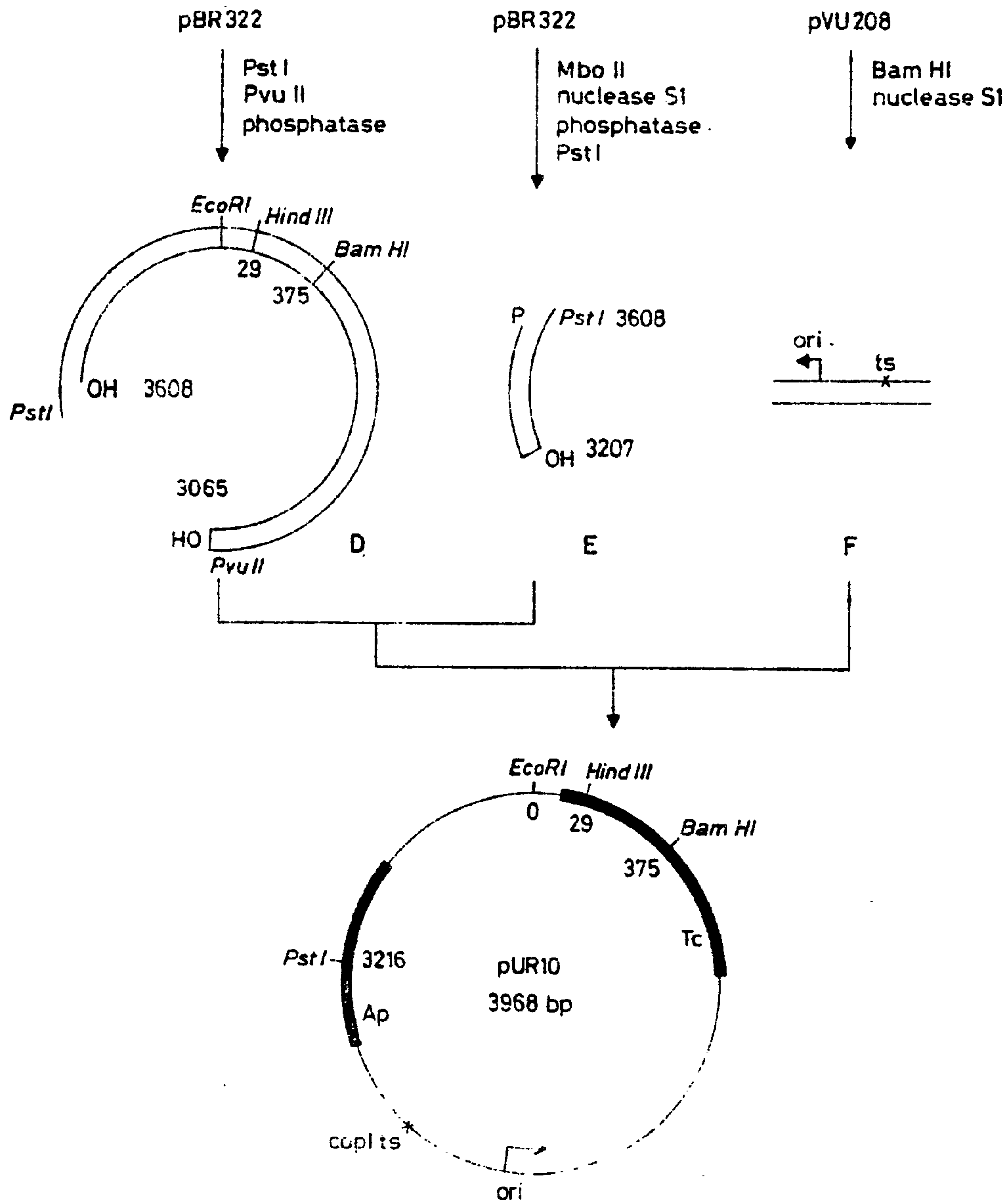
pBR322
Pst I
Pvu II
phosphatase
pBR322
Mbo II
nuclease S1
phosphatase
Pst I
pVU208
Bam HI
nuclease S1
EcoRI
Hind III
Bam HI
29
375
PstI
OH
3608
3065
HO
PvuII
D
P
PstI 3608
OH 3207
E
ori
ts
F
EcoRI
Hind III
0
29
Bam HI
375
Tc
PstI
3216
pUR10
3968 bp
Ap
copI ts
ori

Fig. 13

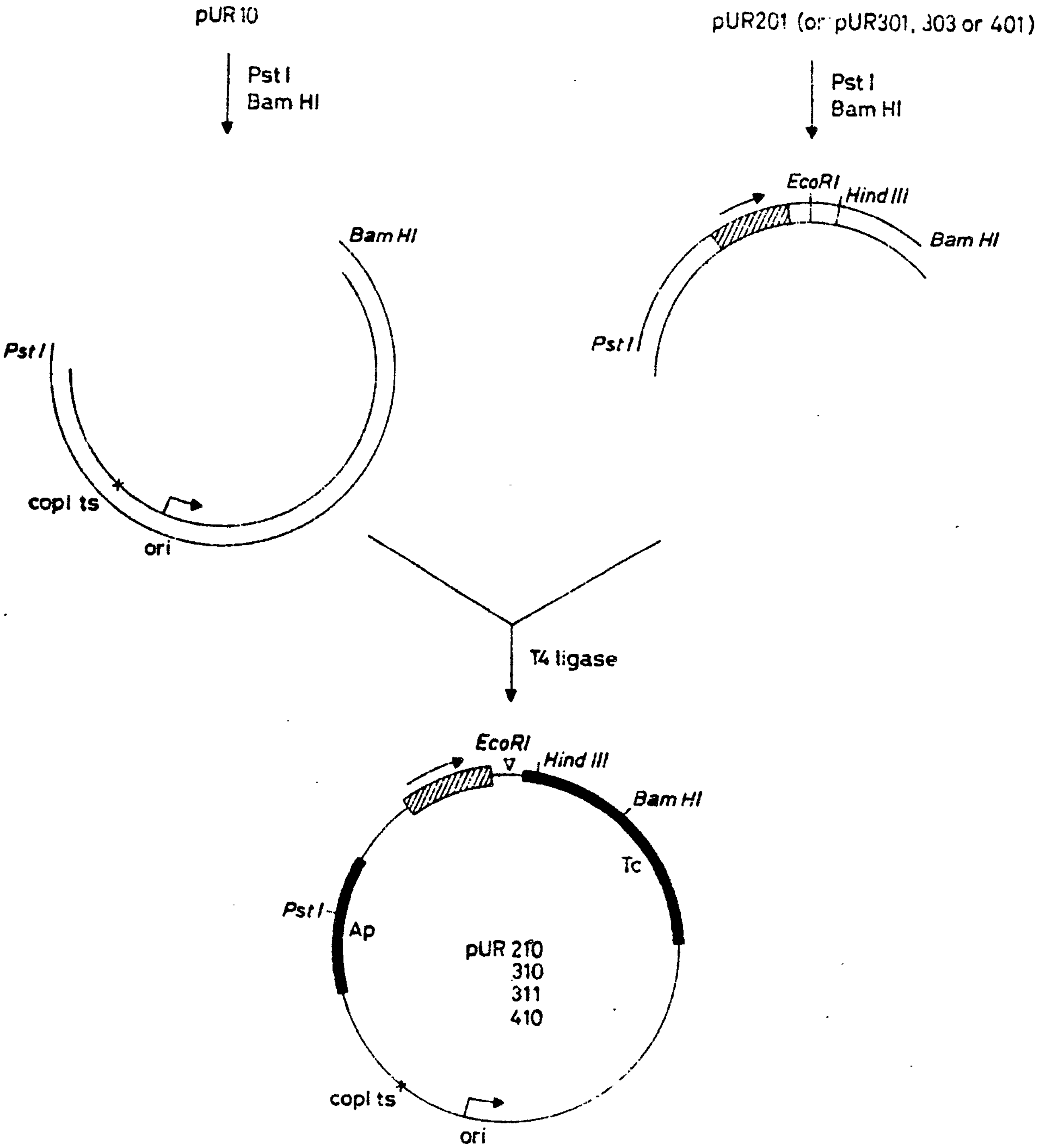
pUR10
Pst I
Bam HI
pUR201 (or pUR301, 303 or 401)
Pst I
Bam HI
Bam HI
Pst I
copl ts
ori
EcoRI
Hind III
Bam HI
Pst I
T4 ligase
EcoRI
Hind III
Bam HI
Tc
Pst I
Ap
pUR 210
310
311
410
copl ts
ori

Fig. 14

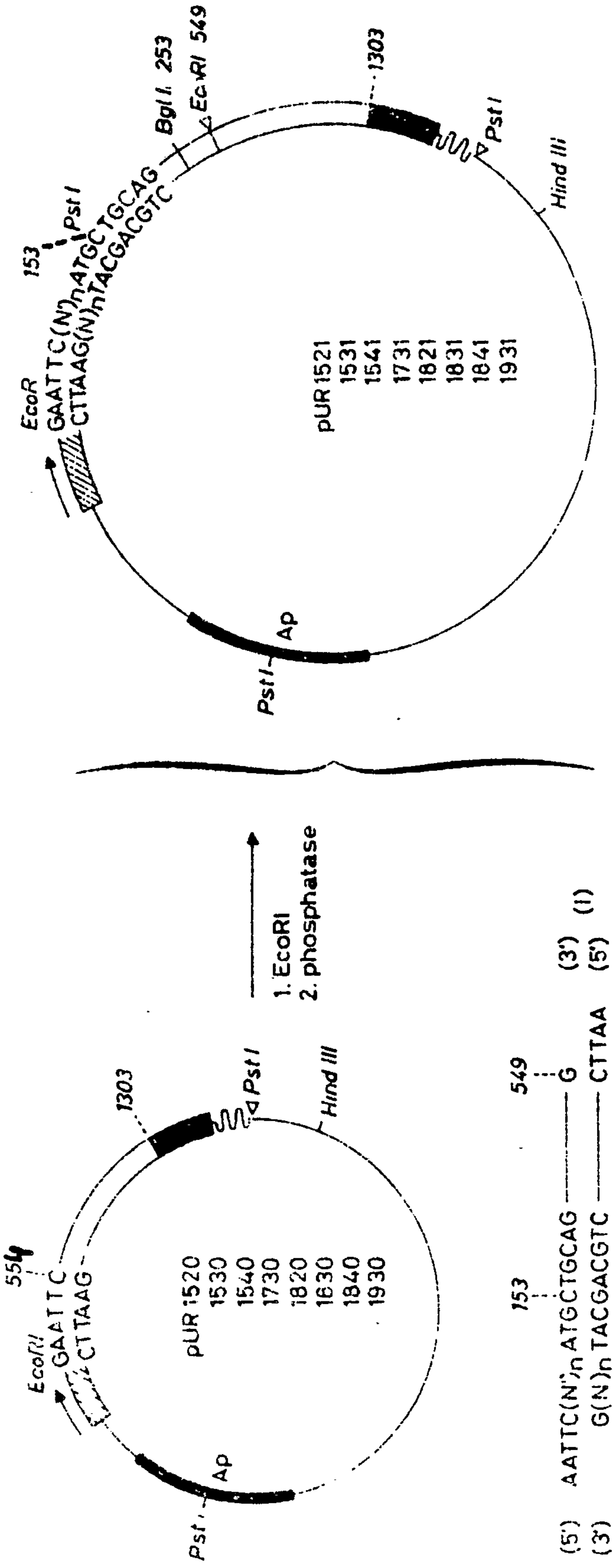
pUR1521
1531
1541
1731
1821
1831
1841
1931
EcoR
GAATTC(N')nATGCTGCAG
CTTAAG(N)nTACGACGTC
153
Pst I
Bgl I. 253
EcoRI 549
1303
Pst I
Hind III
Ap
Pst I
1. EcoRI
2. phosphatase
pUR1520
1530
1540
1730
1820
1630
1840
1930
EcoRI
GAATTC
CTTAAG
1303
Pst I
Hind III
Ap
Pst
(5') AATTC(N')n ATGCTGCAG G (3')
(3') G(N)n TACGACGTC CTTAA (5')
153
549
(I)

Fig. 15

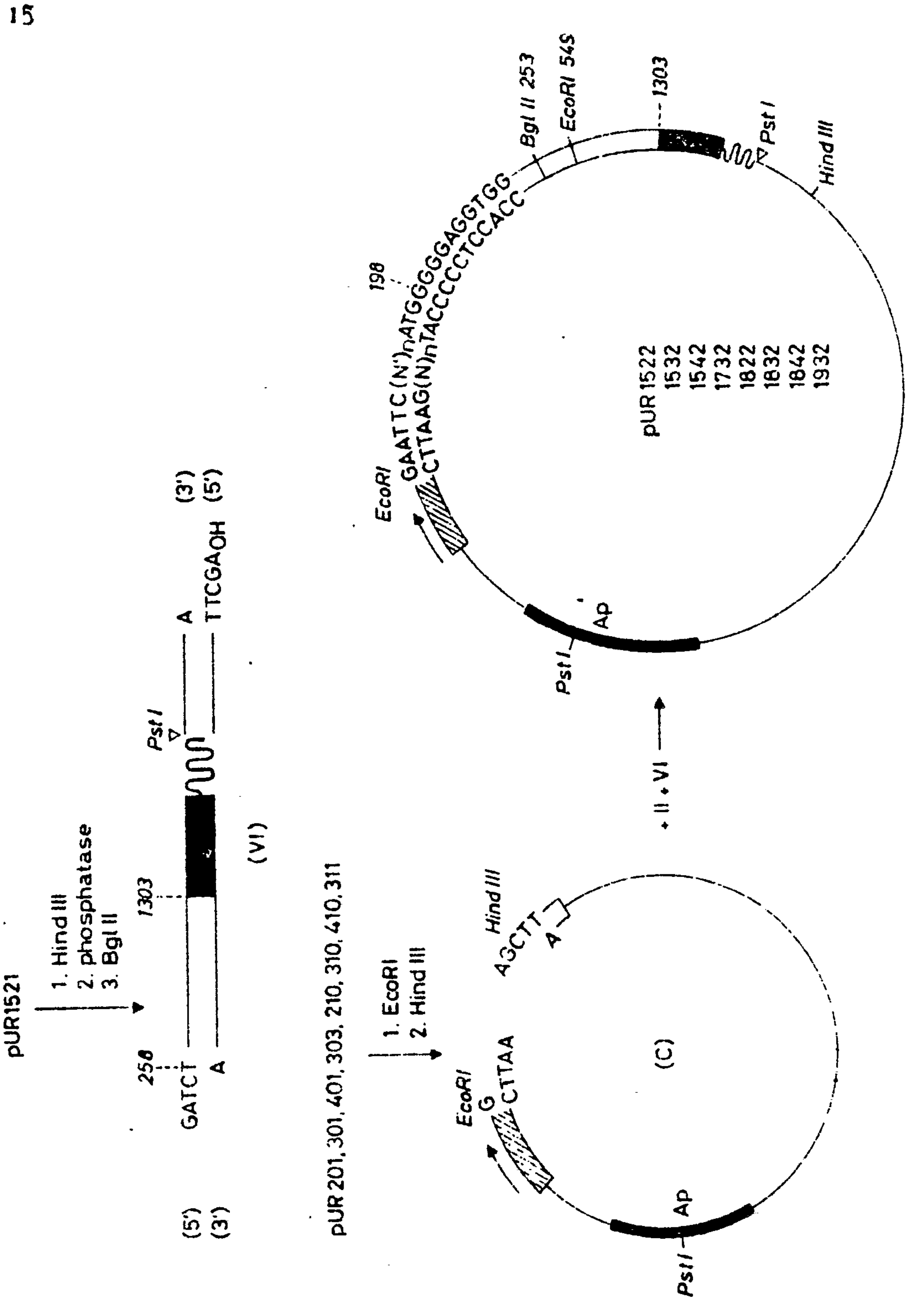
pUR1521
1. Hind III
2. phosphatase
3. Bgl II
258
1303
(5') GATCT A (3')
(3') A TTCGA$_{OH}$ (5')
Pst I
(VI)
pUR 201, 301, 401, 303, 210, 310, 410, 311
1. EcoRI
2. Hind III
EcoRI
G
CTTAA
Hind III
AGCTT
A
(C)
Ap
Pst I
+ II + VI
EcoRI
GAATTC(N')$_n$ATGGGGGAGGTGG
CTTAAG(N)$_n$TACCCCCTCCACC
198
Bgl II 253
EcoRI 545
1303
Pst I
Hind III
pUR 1522
1532
1542
1732
1822
1832
1842
1932
Ap
Pst I

Fig. 16

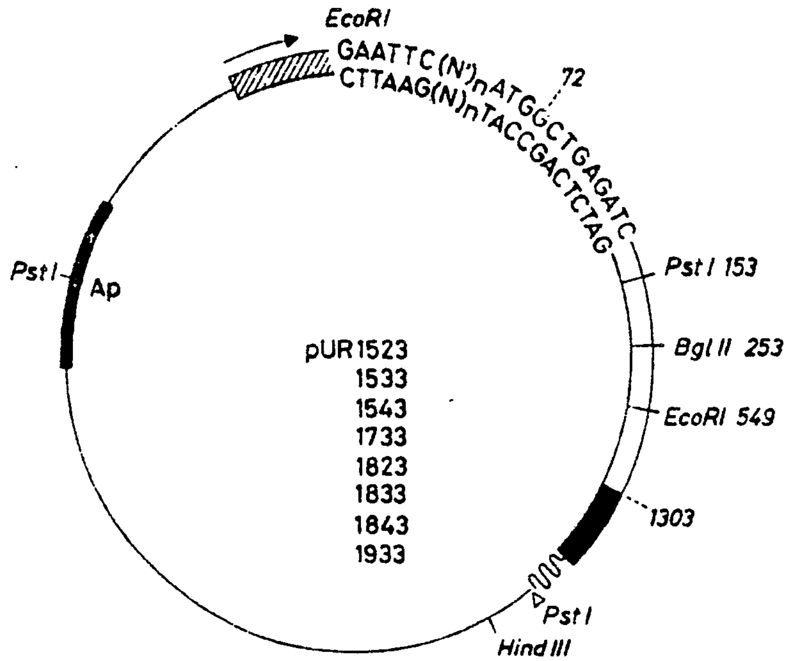
EcoRI
GAATTC(N')nATGGCTGAGATC
CTTAAG(N)nTACCGACTCTAG
72
PstI 153
BglII 253
EcoRI 549
1303
PstI
HindIII
PstI
Ap
pUR1523
1533
1543
1733
1823
1833
1843
1933

Fig. 17

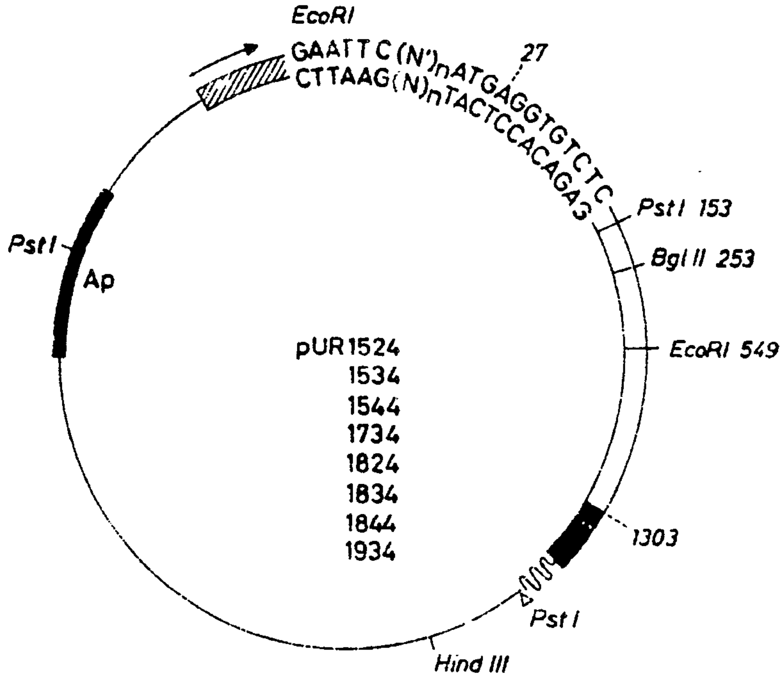
EcoRI
GAATTC(N')nATGAGGTGTCTC
CTTAAG(N)nTACTCCACAGAG
27
PstI 153
BglII 253
EcoRI 549
1303
PstI
HindIII
PstI
Ap
pUR1524
1534
1544
1734
1824
1834
1844
1934

Fig. 18

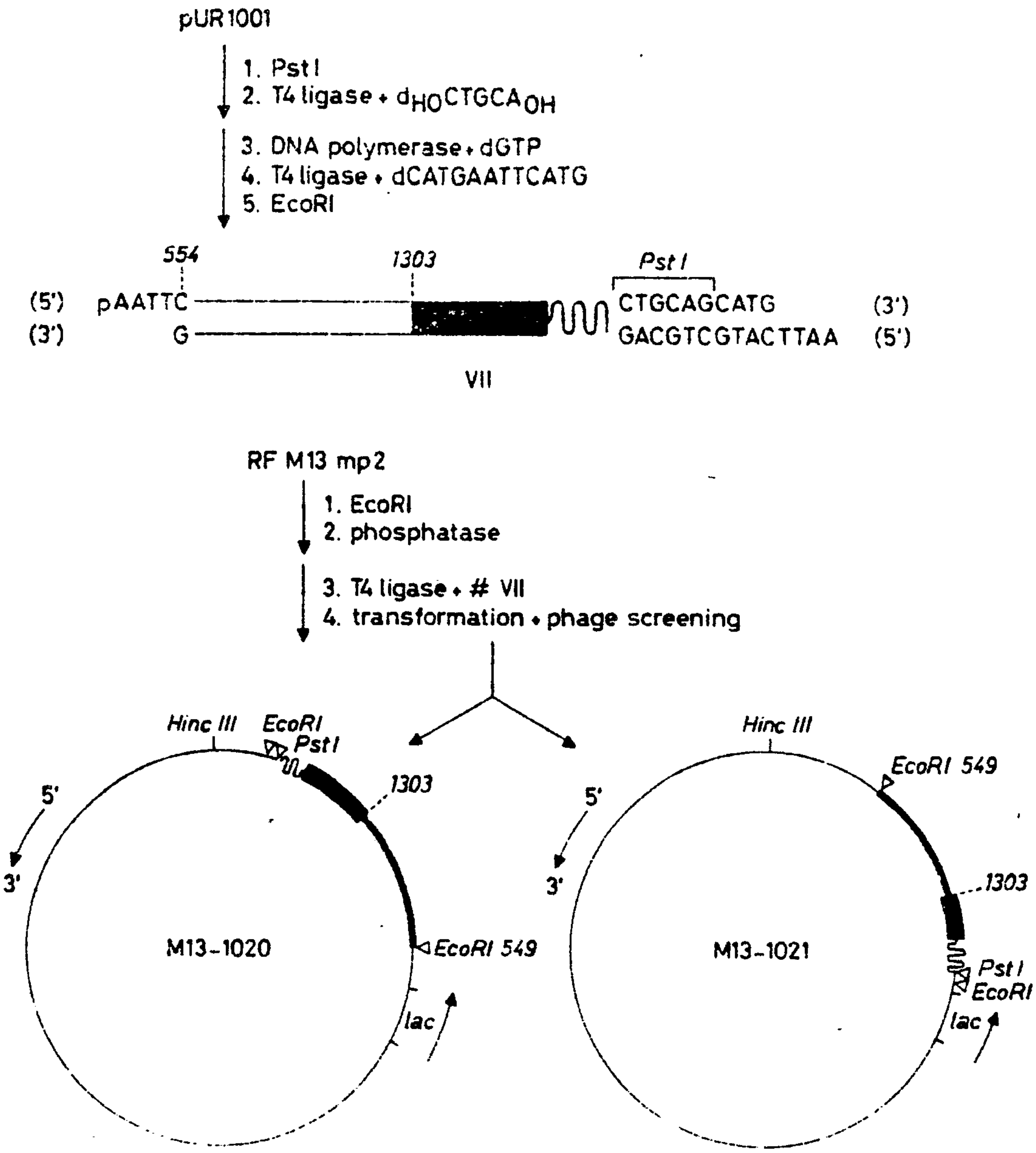
pUR1001
1. PstI
2. T4 ligase + dHOCTGCAOH
3. DNA polymerase + dGTP
4. T4 ligase + dCATGAATTCATG
5. EcoRI
554
1303
PstI
(5') pAATTC
CTGCAGCATG (3')
(3') G
GACGTCGTACTTAA (5')
VII
RF M13 mp2
1. EcoRI
2. phosphatase
3. T4 ligase + # VII
4. transformation + phage screening
HincIII
EcoRI
PstI
1303
5'
3'
M13-1020
EcoRI 549
lac
HincIII
EcoRI 549
5'
3'
1303
M13-1021
PstI
EcoRI
lac

Fig. 18 cont'd

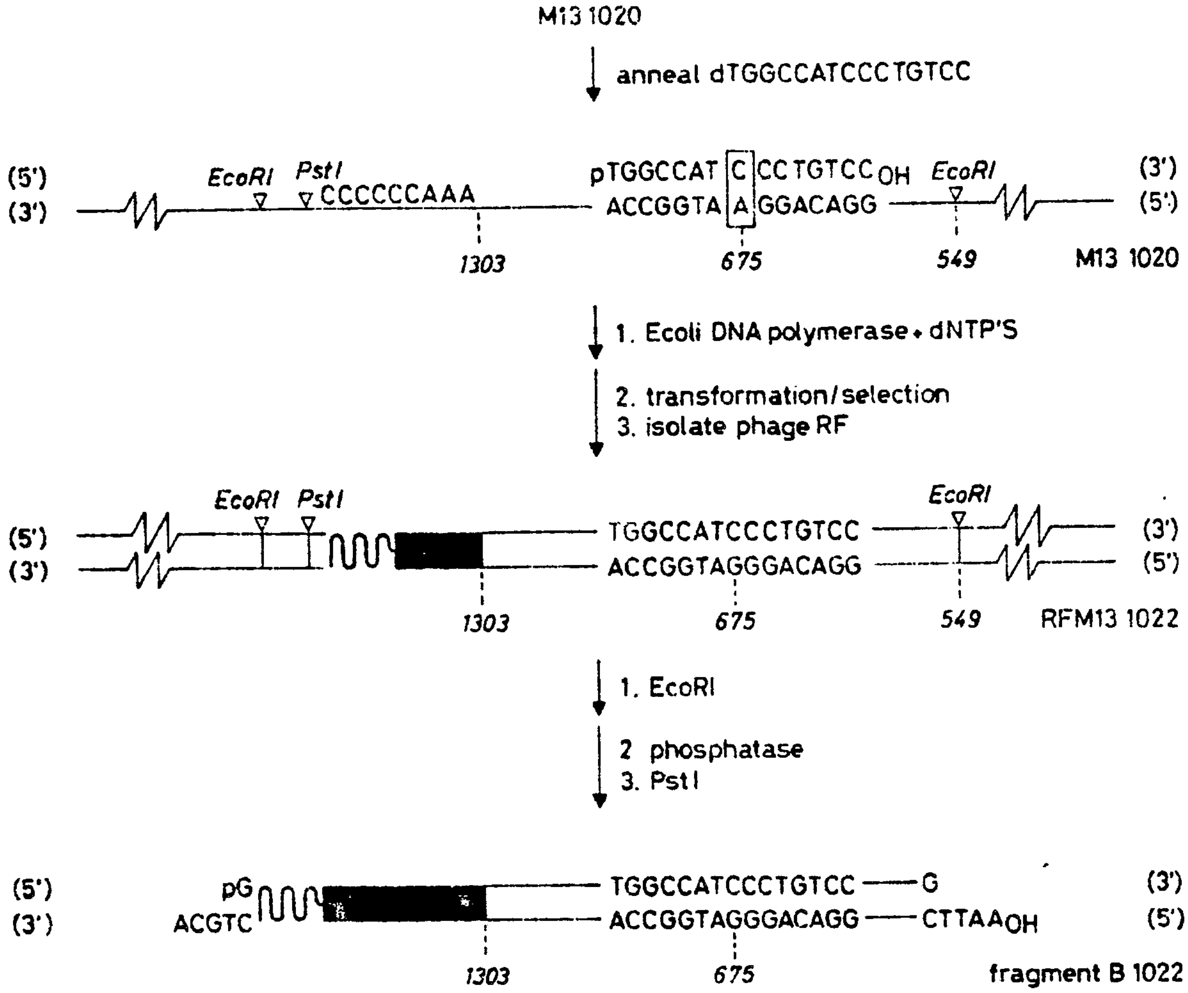
M13 1020
anneal dTGGCCATCCCTGTCC
(5')
(3')
EcoRI
PstI
CCCCCCAAA
pTGGCCAT C CCTGTCC OH
ACCGGTA A GGACAGG
EcoRI
(3')
(5')
1303
675
549
M13 1020
1. Ecoli DNA polymerase + dNTP'S
2. transformation/selection
3. isolate phage RF
EcoRI
PstI
EcoRI
(5')
(3')
TGGCCATCCCTGTCC
ACCGGTAGGGACAGG
(3')
(5')
1303
675
549
RFM13 1022
1. EcoRI
2 phosphatase
3. PstI
(5')
(3')
pG
ACGTC
TGGCCATCCCTGTCC —— G
ACCGGTAGGGACAGG —— CTTAA OH
(3')
(5')
1303
675
fragment B 1022

ATGWGZTGKX TYGTLGTLX TYX TYGCLGTLTTKGCLX TYQRSCAJGGLGCLGAJATMACL
WGZATMCCLX TYTAKAAJGGLAAJQRSX TYWGZAAJGCLX TYAAJGAJCAKGGLX TYX TY
GAJGAKTTKX TYCAJAAJCAJCAJTAKGGLATMQRSQRSAAJTAKQRSGGLTTKGGLGAJ
GTLGCLQRSGTLCCLX TYACLAAKTAKX TYGAKQRSCAJTAKTTKGGLAAJATMTAKX TY
GGLACLCCLCCLCAJGAJTTKACLGTLX TYTTKGAKACLGGLQRSQRSGAKTTKTGGGTL
CCLQRSATMTAKTGKAAJQRSAAKGCLTGKAAJAAKCAKCAJWGZTTKGAKCCLWGZAAJ
QRSQRSACLTTKCAJAAKX TYGGLAAJCCLX TYQRSATMCAKTAKGCIACLGGLQRSATG
CAJGGLATMX TYGGLTAKGAKACLGTLACLGTLQRSAAKATMGTLGAKATMCAJCAJACL
GTLGGLX TYQRSACLCAJGAJCCLGGLGAKGTLTTKACLTAKGCLGAJTTKGAKGGLATM
X TYGGLATGGCLTAKCCLQRSX TYGCLQRSGAJTAKQRSATMCCLGTLTTKGAKAAKATG
ATGAAKQRSCAKX TYGTLGCLCAJGAKX TYTTKQRSGTLTAKATGGAKWGZAAKGGLCAJ
GAJQRSATGX TYACLX TYGGLGCLATMGAKCCLQRSTAKTAKACLGGLQRSX TYCAKTGG
GTLCCLGTLACLGTLCAJCAJTAKTGGCAJTTKACLGTLGAKQRSGTLACLATMQRSGGL
GTLGTLGTLGCLTGKGAJGGLGGLTGKCAJGCLATMX TYGAKACLGGLACLQRSAAJX TY
GTLGGLCCLQRSQRSGAKATMX TYAAKATMCAJCAJGCLATMGGLGCLACLCAJAAKCAJ
TAKGGLGAJTTKGAKATMGAKTGKCAKAAKX TYQRSTAKATGCCLACLGTLGTLTTKGAJ
ATMAAKGGLAAJATGTAKCCLX TYACLCCLQRSGCLTAKACLQRSCAJGAKCAJGGLTTK
TGKACLQRSGGLTTKCAJQRSGAJAAKCAKQRSCAJAAJTGGATMX TYGGLGAKGTLTTK
ATMWGZGAJTAKTAKQRSGTLTTKGAKWGZGCLAAKAAKX TYGTLGGKX TYGCLAAJGCL
ATMTGA

Fig. 19

ATGGCLGAJATMACLWGZATMCCLX TYTAKAAJGGLAAJQRSX TYWGZAAJGCLX TYAAJ
GAJCAKGGLX TYX TYGAJGAKTTKX TYCAJAAJCAJCAJTAKGGLATMQRSQRSAAJTAK
QRSGGLTTKGGLGAJGTLGCLQRSGTLCCLX TYACLAAKTAKX TYGAKQRSCAJTAKTTK
GGLAAJATMTAKX TYCGLACLCCLCCLCAJCAJTTYACLGTLX TYTTKGAKACLGGLQRS
QRSCAKTTKTGGGTLCCLQRSATMTAKTGKAAJQRSAAKGCLTGKAAJAAKCAKCAJWGZ
TTKGAKCCLWGXAAJQRSQRSACLTTKCAJAAKX TYGGLAAJCCLX TYQRSATMCAKTAK
GGLACLGGLQRSATGCAJGGLATMX TYGGLTAKGAKACLGTLACLGTLQRSAAKATMGTL
GAKATMCAJCAJACLGTLGGLX TYQRSACLCAJGAJCCLGGLGAKGTLTTKACLTAKGCL
GAJTTKGAKGGLATMX TYGGLATGGCLTAKCCLQRSX TYGCLQRSGAJTAKQRSATMCCL
GTLTTKGAKAAKATGATGAAKQRSCAKX TYGTLGCLCAJGAKX TYTTKQRSGTLTAKATG
GAKWGZAAKGGLCAJGAJQRSATGX TYACLX TYGGLGCLATMGAKCCLQRSTAKTAKACL
GGLQRSX TYCAKTGGGTLCCLGTLACLGTLCAJCAJTAKTGGCAJTTKACLGTLGAKQRS
GTLACLATMQRSGGLGTLGTLGTLGCLTGKGAJGGLGGLTGKCAJGCLATMX TYGAKACL
GGLACLQRSAAJX TYGTLGGLCCLQRSQRSGAKATMX TYAAKATMCAJCAJGCLATMGGL
GCLACLCAJAAKCAJTAKGGLGAJTTKGAKATMGAKTGKGAKAAKX TYQRSTAKATGCCL
ACLGTLGTLTTKGAJATMAAKGGLAAJATGTAKCCLX TYACLCCLQRSGCLTAKACLQRS
CAJGAKCAJGGLTTKTGKACLQRSGGLTTKCAJQRSGAJAAKCAKQRSCAJAAJTGGATM
X TYGGLGAKGTLTTKATMWGZGAJTAKTAKQRSGTLTTKGAKWGZGCLAAKAAKX TYGTL
GGKX TYGCLAAJGCLATMTGA

Fig. 20

ATGX TYCAJAAJCAJCAJTAKGGLATMQRSQRSAAJTAKQRSGGLTTKGGLGAJGTLGCL
QRSGTLCCLX TYACLAAKTAKX TYGAKQRSCAJTAKTTKGGLAAJATMTAKX TYGGLACL
CCLCCLCAJGAJTTKACLGTLX TYTTKGAKACLGGLQRSQRSGAKTTKTGGGTLCCLQRS
ATMTAKTGKAAJQRSAAKGCLTGKAAJAAKCAKCAJWGZTTKGAKCCLWGKAAJQRSQRS
ACLTTKCAJAAKX TYGGLAAJCCLX TYQRSATMCAKTAKGGLACLGGLQRSATGCAJGGL
ATMX TYGGLTAKGAKACLGTLACLGTLQRSAAKATMGTLGAKATMCAJCAJACLGTLGGL
X TYQRSACLCAJGAJCCLGGLGAKGTLTTKACLTAKGCLCAJTTKGAKGGLATMX TYGGL
ATGGCLTAKCCLQRSX TYGCLQRSGAJTAKQRSATMCCLCTLTTKGAKAAKATGATGAAK
QRSCAKX TYGTLGCLCAJGAKX TYTTKQRSGTLTAKATGGAKWGZAAKGGLCAJGA QRS
ATGX TYACLX TYGGLCCLATMGAKCCLQRSTAKTAKACLGGLQRSX TYCAKTGGGTLCCL
GTLACLGTLCAJCAJTAKTGGCAJTTKACLGTLGAKQRSGTLACLATMQRSGGLGTLGTL
GTLGCLTGKGAJGGLGGLTGKCAJGCLATMX TYGAKACLGGLACLQRSAAJX TYGTLGGL
CCLQRSQRSGAKATMX TYAAKATMCAJCAJGCLATMGGLGCLACLCAJAAKCAJTAKGGL
GAJTTKGAKATMGAKTGKCAKAAKX TYQRSTAKATGCCLACLGTLGTLTTKGAJATMAAK
CGLAAJATGTAKCCLX TYACLCCLQRSGCLTAKACLQRSCAJGAKCAJGGLTTKTGKACL
QRSGGLTTKCAJQRSGAJAAKCAKQRSCAJAAJTGGATMX TYGGLGAKGTLTTKATMWGZ
GAJTAKTAKQRSGTLTTKGAKWGZGCLAAKAAKX TYGTLGGKX TYGCLAAJGCLATMTGA

Fig. 21

ATGGGLCAJGTLGCLQRSGTLCCLX TYACLAAKTAKX TYGAKQRSCAJTAKTTKGGLAAJ
ATMTAKX TYGGLACLCCLCCLCAJCAJTTKACLGTLX TYTTKGAKACLGGLQRSQRSGAK
TTKTGGGTLCCLQRSATMTAKTGKAAJQRSAAKGGLTGKAAJAAKCAKCAJWGZTTKGAK
CCLWCKAAJQRSQRSACLTTKCAJAAKX TYGGLAAJCCLX TYQRSATMCAKTAKGGLACL
GGLQRSATGCAJGGLATMX TYGGLTAKGAKACLGTLACLGTLQRSAAKATMGTLGAKATM
CAJCAJACLCTLGGLX TYQRSACLCAJGAJCCLGGLGAKGTLTTKACLTAKGCLCAJTTK
GAKGCLATMX TYGGLATGGCLTAKCCLQRSX TYGCLQRSGAJTAKQRSATMCCLGTLTTK
GAKAAKATGATGAAKQRSCAKX TYGTLGCLCAJGAKX TYTTKQRSGTLTAKATGGAKWGZ
AAKGGLCAJGAJQRSATGX TYACLX TYGGLGCLATMGAKCCLQRSTAKTAKACLGCLQRS
X TYCAKTGGGTLCCLGTLACLGTLCAJCAJTAKTGGCAJTTKACLGTLGAKQRSGTLACL
ATMQRSGGLGTLGTLGTLGCLTGKGAJGGLGGLTGKCAJGCL ATMX TYGAKACLGGLACL
QRSAAJX TYGTLGGLCCLQRSQRSGAKATMX TYAAKATMCAJCAJGCLATMCGLGCLACL
CAJAAKCAJTAKGGLGAJTTKGAKATMGAKTGKGAKAAKX TYQRSTAKATGCCLACLGTL
GTLTTKGAJATMAAKGGLAAJATGTAKCCLX TYACLCCLQRSGCLTAKACLQRSCAJGAK
CAJGGLTTKTGKACLQRSGGLTTKCAJQRSGAJAAKCAKQRSCAJAAJTGGATMX TYGGL
GAKGTLTTKATMWGZGAJTAKTAKQRSGTLTTKGAKWGZGCLAAKAAKX TYGTLGGKX TY
GCLAAJGCLATMTGA

Fig. 22